# EUROPÄISCHE PATENTSCHRIFT

(11) **EP 2 291 158 B1**
(45) Veröffentlichungstag und Bekanntmachung des Hinweises auf die Patenterteilung: **23.11.2016**
(21) Anmeldenummer: 09765573.2
(22) Anmeldetag: 12.06.2009
(51) Int. Cl.: A61B 46/00, A61F 13/51

(54) **OP-ABDECKUNG MIT INTEGRIERTER FLÜSSIGKEITSBARRIERE**
SURGICAL DRAPE WITH INTEGRATED LIQUID BARRIER
CHAMP OPÉRATOIRE AVEC BARRIERE AUX LIQUIDES INTÉGRÉ

(30) Priorität: 18.06.2008 DE 102008029051
(43) Veröffentlichungstag der Anmeldung: 09.03.2011
(73) Patentinhaber: Paul Hartmann Aktiengesellschaft, 89522 Heidenheim (DE)
(72) Erfinder: HOFFMANN, Thomas, 89522 Heidenheim-Oggenhausen (DE)
(86) Internationale Anmeldenummer: PCT/EP2009/004230
(87) Internationale Veröffentlichungsnummer: WO 2009/152993

(56) Entgegenhaltungen:
- US-A- 5 222 507
- US-A1- 2007 135 784

## Beschreibung

Die Erfindung betrifft eine wegwerfbare OP-Abdeckung mit absorbierenden Bereichen. Derartige OP-Abdeckungen weisen ein flüssigkeitsundurchlässiges Basisabdeckmaterial mit einer in Gebrauch zum Patienten gerichteten Unterseite und einer Oberseite auf, wobei die Oberseite eine absorbierende Schicht, einen ersten absorbierenden Bereich und mindestens einen zweiten absorbierenden Bereich aufweist. Der zweite absorbierende Bereich wird dabei von einem Flüssigkeiten absorbierenden und flüssigkeitsdurchlässigen Materialabschnitt mit Randkanten und angrenzenden Randbereichen überfangen. Die OP-Abdeckung als solche erstreckt sich in einer Längsrichtung LR zwischen einem oberen Ende und einem unteren Ende.

Wegwerfbare OP-Abdeckungen umfassen für den einmaligen Gebrauch vorgesehene Abdeckungen für jegliche Art von Operationen.
OP-Abdeckungen dienen der sterilen Abdeckung des Patienten während eines chirurgischen Eingriffes. Zu wesentlichen Eigenschaften einer OP-Abdeckung gehören Barrierefunktion in Hinblick auf Keimdichtigkeit und Widerstand gegen Flüssigkeitspenetration, ebenso ausreichende Materialfestigkeit (Berst - und Zugfestigkeit), des Weiteren möglichst geringe Abgabe von Partikeln an die Umgebung (Fremdstaubbelastung, Linting) und dazu mikrobiologische Reinheit. All diese Anforderungen sind in der EN 13795 Teil 1 2003, Teil 2 2005, Teil 3 2006 beschrieben.

Zusätzlich zu den oben genannten normativ festgelegten Anforderungen wird in der Praxis auch die Anforderung an ein ausreichend gutes Flüssigkeitsmanagement durch die OP-Abdeckung während des chirurgischen Eingriffes gestellt. In der Praxis sollte eine OP-Abdeckung für flüssigkeitsarme bis hin zu sehr flüssigkeitsreiche, oft auch mit schwallartigem Flüssigkeitsaufkommen verbundene chirurgische Eingriffe, geeignet sein.

Es sind bereits OP-Abdeckungen bekannt, welche Verbesserungsmaßnahmen zum Sammeln von während der Operation auftretenden und aus dem Operationsfeld heraustretenden Flüssigkeit zeigen.
Das Flüssigkeitsabsorptionsvermögen der Basisabdeckmaterialien ist sehr unterschiedlich. Wird die Absorption als nicht ausreichend betrachtet, so können die OP-Abdeckungen bereichsweise absorbierende Verstärkungen aufweisen.
So zeigt beispielsweise US 3,902,484 eine OP-Abdeckung aus einem Basisabdeckmaterial mit einem auf der Oberseite angebrachten Materialabschnitt bestehend aus einer an diese Oberseite angrenzenden Folienlage und einer absorbierenden Schaumstofflage.

So sind auch OP-Abdeckungen mit einer absorbierenden Oberseite und einem darauf mittels streifenförmigen Hotmeltkleberauftrag fixierten einlagigen absorbierenden Pad bekannt.

OP-Abdeckungen werden in der Praxis auch zumeist derart eingesetzt, dass die OP-Abdeckung im Gebrauch sich in einer Schräglage befindet. Trotz der Absorptionskapazität eines auf das Basisabdeckmaterial angebrachten absorbierenden Materialabschnitts und auch dessen lateraler Verteilungskapazität wandert bei länger andauernden chirurgischen Eingriffen die absorbierte Flüssigkeit entsprechend der Schwerkraft folgend nach unten zum unteren Rand des absorbierenden Materialabschnittes und weiter dem Basisabdeckmaterial folgend zum unteren Rand des Basisabdeckmaterials. Dort beginnt es dann, aus der OP-Abdeckung zu tropfen und es kommt zu einer Kontamination der darunter befindenden Bereiche und insbesondere u.a. auch des Bodens des Operationssaales.

Um dieser Problematik entgegen zu treten, werden bei besonders flüssigkeitsreichen Eingriffen mit oftmals schwallartigem Flüssigkeitsaufkommen die OP-Abdeckungen zusätzlich mit einem separat angesetzten, speziell konstruierten Flüssigkeitsauffangbeutel ausgestattet. Der Nachteil der angefügten Beutel liegt zumeist in der sperrigen Konstruktion, welche den Chirurg während seiner Arbeit behindert.

Es sind auch integral angebrachte Flüssigkeitsauffangtaschen bekannt. So sind aus WO 02/41800 A2 und EP 0 631 760 B1 OP-Abdeckungen bekannt, welche durch Umfalten eines Teils eines Materialabschnittes und Fixierens auf sich selbst mittels Hook/Loop-Mechanismus bzw. sonstiger wieder lösbarer Fixiermittel eine zum Operationsfeld geöffnete Rinne aufweisen. US 4,089,331 und US 3,791,382 zeigen ebenso eine OP-Abdeckung mit einem um das OP-Fenster angeordneten Verstärkungsabschnitt, aus welchem durch mehrmalige Faltung des Materialabschnitts eine zum OP-Fenster geöffnete Auffangrinne bereitgestellt wird.

US 2007/0135784 A1 zeigt eine OP-Abdeckung mit einer mit superabsorbierenden Material gefüllten Taschenstruktur, wobei die Taschenstruktur aus einer flüssigkeitsdurchlässigen absorbierenden oberen Lage und einer flüssigkeitsundurchlässigen unteren Lage, welche mittels einer abdichtenden Linie miteinander verbunden sind, gebildet ist.

US 5,464,024 zeigt eine wiederverwendbare OP-Abdeckung mit einer das OP-Fenster umgebenden und in Richtung OP-Fenster offenen Flüssigkeitsauffangrinne. Diese Auffangrinne wird durch die Faltung eines um das OP-Fenster angeordneten flüssigkeitsabweisenden Materialabschnittes in Richtung OP-Fenster und dessen partielle Fixierung auf sich selbst erhalten.

WO 2006/0389948 A1 beschreibt eine OP-Abdeckung bestehend aus einem mittleren Tuchabschnitt mit OP-Fenster und zwei weiteren Tuchabschnitten, welche an den jeweiligen Längskanten des mittleren Tuchabschnittes befestigt werden. Die Enden des mittleren Tuchabschnittes werden dann eingeschlagen und auf demselben befestigt, wodurch sich eine Manschette zum Auffangen von Flüssigkeiten bildet.

Der Erfindung liegt die Aufgabe zu Grunde eine OP-Abdeckung der vorgenannten Art dahingehend zu verbessern, dass eine ausreichende Aufnahmekapazität für Flüssigkeiten und eine ausreichende Retardierung des Flüssigkeitsaustrittes gleichzeitig bei einer einfachen und preiswerten Konstruktion der OP-Abdeckung bereitgestellt wird.

Diese Aufgabe wird erfindungsgemäß gelöst durch eine OP-Abdeckung mit den Merkmalen gemäß Anspruch 1.

Zur Lösung dieser Aufgabe wird also eine wegwerfbare OP-Abdeckung der eingangs genannten Art vorgeschlagen, bei der der absorbierende Materialabschnitt in den Randbereichen vollumfänglich auf der Oberseite des Basisabdeckmaterials unlösbar fixiert ist und wobei der absorbierende Materialabschnitt außerhalb der Randbereiche im Wesentlichen nicht mit dem Basisabdeckmaterial verbunden ist, derart, dass zwischen dem absorbierenden Materialabschnitt und dem flüssigkeitsundurchlässigen Basisabdeckmaterial eine Tasche gebildet ist. Zumindest einer der Randbereiche des absorbierenden Materialabschnitts weist zumindest abschnittsweise eine Flüssigkeitsbarriere auf, wobei die Flüssigkeitsbarriere durch ein Fügemittel gebildet ist, welches den Materialabschnitt unlösbar auf der Oberseite des Basisabdeckmaterials fixiert. Hierbei wird das Verständnis zugrunde gelegt, dass der zweite absorbierende Bereich durch die flächenhafte Erstreckung des absorbierenden Materialabschnitts und auch durch die vom absorbierenden Materialabschnitt überfangene flächenhafte Erstreckung des darunter liegenden Basisabdeckmaterials gebildet wird. Diejenige verbleibende Fläche der Oberseite des Basisabdeckmaterials, die nicht durch einen absorbierenden Materialabschnitt abgedeckt ist, bildet den ersten absorbierenden Bereich.

"Außerhalb der Randbereiche im Wesentlichen nicht mit dem Basisabdeckmaterial verbunden" heißt, dass die außerhalb der fixierten Randbereiche verbliebene Fläche des absorbierenden Materialabschnitts zusätzliche Bindungen des absorbierenden Materialabschnitts mit dem Basisabdeckmaterial mit einem Flächenanteil von höchstens 10%, insbesondere von höchstens 5%, weiter insbesondere von höchstens 2,5% bezogen auf die Gesamtfläche des absorbierenden Materialabschnittes aufweist.

"Vollumfänglich" wird dabei verstanden als der gesamte Umfang, welcher durch die Begrenzungslinie eines ebenen Flächengebildes beschrieben ist. Das Flächengebilde kann dabei die regelmäßige flächenhafte Erstreckung eines Polygons, wie beispielsweise eines Vierecks, eines Rechtecks, einnehmen. Ebenso kann das ebene Flächengebilde durch eine Begrenzungslinie beschrieben sein, welche ausgehend von einer vom Betrachter primär wahrgenommenen äußeren Kontur des Flächengebildes eine nach radial innen verlaufende Einbuchtung aufweist, so z.B. in Form einer schlitzförmigen Ausnehmung bei einem ansonsten rechteckigen Flächengebilde. Der Umfang ist die Summe aller Begrenzungslinien.

Als "Längsrichtung" wird dabei jegliche flächenhafte Erstreckung zwischen zwei sich gegenüber liegenden Enden der OP-Abdeckung verstanden. Das obere und untere Ende der OP-Abdeckung wird durch die im Gebrauch der OP-Abdeckung vorgesehene Anordnung in Relation zur Bodenebene festgelegt.
Ein oberes und ein unteres Ende in Längsrichtung der OP-Abdeckung werden durch den vorgesehenen Einsatzzweck der jeweiligen OP-Abdeckung bestimmt.
Die für den jeweiligen Einsatzzweck bevorzugte Anordnung der OP-Abdeckung in Längsrichtung wird dem Anwender, also dem medizinischen Personal, vorteilhafterweise durch Orientierungshilfen angezeigt.
Als Orientierungshilfen sind dabei verschiedene Ausführungen denkbar, so beispielsweise in Form von Symbolen, Grafiken, Pfeilen mit Richtungsangabe oder auch als schematische Darstellung von menschlichen Körperteilen wie Füße oder Kopf, welche dem Anwender die die Orientierung von einem oberen und unteren Ende der OP-Abdeckung suggerieren. Weiterhin vorteilhafterweise sind als Orientierungshilfe für die Erkennung eines oberen Endes und eines unteren Endes herstellerseitig entlang des oberen Randes der OP-Abdeckung, und dabei vorzugsweise auf der Unterseite des Basisabdeckmaterials, Fixierungsmittel zur Fixierung der OP-Abdeckung am Patienten oder auf bereits andere vorlegte Abdeckungen angebracht.

Mit der Erfindung wurde erkannt, dass eine derartige Konstruktion einer OP-Abdeckung wesentliche Vorteile mit sich bringt.

Zum einen kann durch die Kombination eines Flüssigkeiten absorbierenden und durchlässigen Materialabschnitts mit einer direkt darunter angeordneten absorbierenden Oberseite des Basisabdeckmaterials, ohne dass eine flüssigkeitsundurchlässige Zwischenlage dazwischen geschaltet ist, die vom absorbierenden Materialabschnitt primär aufgenommene Flüssigkeit auch an das Basisabdeckmaterial weitergeleitet und von diesem aufgenommen und verteilt werden. Damit kann ein optimiertes Flüssigkeitsaufnahmevermögen in den möglicherweise großen Flüssigkeitsmengen ausgesetzten kritischen Bereichen einer OP-Abdeckung bereitgestellt werden. Die Saugfähigkeit von Materialien kann üblicherweise über ihre Materialzusammensetzung und deren gezielte Auswahl und/oder ihre Dicke und/oder über ein hohes Flächengewicht erreicht werden. Üblicherweise werden deshalb separat gestaltete, dicke Verstärkungsmaterialien auf eine OP-Abdeckung aufgebracht. Durch die Kombination der absorbierenden Schicht des Basisabdeckmaterials, welches die OP-Abdeckung bereits als solche bildet, mit bewusst zonal angeordneten absorbierenden Materialabschnitten können Bereiche mit einem optimierten Flächengewicht bei Materialeinsparung und damit einhergehend Kosteneinsparung bereitgestellt werden.

Zum anderen schafft die im Wesentlichen nur randständige Fixierung des absorbierenden Materialabschnitts auf dem Basisabdeckmaterial zwischen den beiden Materialschichten eine Tasche, in welcher sich in einer Art Glasplatteneffekt, also der Adhäsionskraft zwischen den Materialschichten, zusätzlich Flüssigkeit ansammeln und verteilen kann.

Ebenso wird durch die randständige Fixierung keine unnötige Versteifung der OP-Abdeckung herbeigeführt. Somit wird auch die Drapierfähigkeit der OP-Abdeckung nur in einem äußerst geringen Maße beeinträchtigt. Ebenso erweist es sich als vorteilhaft, dass die Fixierung des absorbierenden Materialabschnitts im Wesentlichen nur randständig erfolgt, da auf diese Weise das durch die flächenhafte Erstreckung des absorbierenden Materialabschnittes bereitgestellte Absorptionsvermögen nur in einem geringen Maße beeinträchtigt wird.
Ebenso wurde erkannt, dass zur Fixierung des absorbierenden Materialabschnitts an das Basisabdeckmaterial Fügemittel eingesetzt werden können, welche gleichzeitig Flüssigkeitsbarrieren in den Randbereichen ausbilden. Die im Wesentlichen nur durch randständige Fixierung des absorbierenden Materialabschnittes an das Basisabdeckmaterial erhaltene integrale Tasche wird zumindest abschnittsweise entlang ihrer Begrenzungslinien abgedichtet. Damit wird eine in die OP-Abdeckung integrierte Auffangvorrichtung, also ein Flüssigkeitssammelreservoir geschaffen, wobei vorteilhaft keine weiteren Materiallagen benötigt werden.

Insgesamt wird bei geringem Konstruktionsaufwand eine OP-Abdeckung mit einem optimierten Flüssigkeitsmanagement erhalten.

Als Fügemittel werden sämtliche Mittel verstanden, welche zur Fixierung von Materialien, also von der Fixierung des absorbierenden Materialabschnittes an das Basisabdeckmaterial, eingesetzt werden.

Besonders vorteilhaft wird das Fügemittel entnommen aus der Gruppe Hotmeltkleber, ein- oder beidseitiges Klebeband, Schweißstellen, dabei insbesondere Ultraschallschweißstellen, thermische Schweißstellen und Kalanderschweißstellen.

Die Fügemittel sind dabei derart gestaltet, dass durch die unlösbare Fixierung des absorbierenden Materialabschnittes in den Randbereichen auf der Oberseite des Basisabdeckmaterials eine Flüssigkeitsbarriere gebildet wird.

Dabei können die Fügemittel selbst aufgrund ihrer Materialeigenschaften bereits Flüssigkeitsbarriereeigenschaften besitzen.

Hotmeltkleber sind nassstabil und weisen hydrophobe Eigenschaften auf. Hotmeltkleber, insbesondere in Form von kontinuierlichen Streifen bilden eine Flüssigkeitsbarriere. Zusätzlich kann der Hotmeltkleber z.T. in die Tiefe der zu verbindenden Materialien eindringen, die Poren verschließen und damit die Barrierewirkung erhöhen.

Ein Klebeband als solches weist eine flüssigkeitsundurchlässige polymere Lage auf, an welche sich ein- oder beidseitig und/oder auch jeweils nur bereichsweise eine adhäsive Beschichtung anschließt, mittels welcher die eigentliche unlösbare Verbindung zwischen absorbierendem Materialabschnitt und dem Basisabdeckmaterial erzeugt wird.

Schweißstellen können mittels verschiedener Verfahren, wie Ultraschall, thermisch, Kalander erzeugt werden. Schweißverfahren wirken dabei direkt auf das dem Schweißverfahren unterworfene Material ein, was an bzw. in den entsprechend behandelten Stellen neben dem Resultat des Zusammenfügens auch zumeist eine Änderung der Materialeigenschaften zur Folge hat (bspw. Umwandeln des loftigen Charakters eines Vlieses in einen folienähnlichen Charakter). Die Bindungsstärke der jeweiligen Schweißstellen kann durch die Wahl der Ultraschallstärke bzw. der Temperatur bzw. des Druckes eingestellt werden.

In einer vorteilhaften Weiterbildung der Erfindung weist die OP-Abdeckung innerhalb des zweiten absorbierenden Bereiches zumindest eine Ausnehmung auf.
Die Ausnehmung kann dabei unterschiedlich gestaltet sein. Hierbei wird das Verständnis zugrunde gelegt, dass eine Ausnehmung eine vollumfänglich durch Material begrenzte Öffnung sein kann. Die Öffnung kann dabei jegliche Kontur aufweisen, so also rund, oval, viereckig, rechteckig oder polygonal mit abgerundeten Ecken sein.
Die Ausnehmungen können dabei jegliche Dimension aufweisen. Vorzugweise haben kreisrunde Öffnungen eine Dimension von 4 - 12 cm, ovale Öffnungen eine Größe von 10 x 14 cm, insbesondere 5 x 7 cm. Öffnungen jeglicher Kontur können durchaus auch eine Größe von 20 x 30 cm einnehmen.

Ebenso kann eine Ausnehmung ausgehend von der vom Betrachter primär wahrgenommenen äußeren Kontur des Flächengebildes als eine Einbuchtung in Richtung Mitte der OP-Abdeckung ausgebildet sein. Die Einbuchtung kann dabei jegliche Kontur aufweisen, so beispielsweise in Form einer schlitzförmigen oder U-förmigen Einbuchtung unter Ausbildung von zwei diese Ausnehmung begrenzenden Materiallappen.
Derartige Einbuchtungen können dabei jegliche Dimension aufweisen. Vorzugsweise können derartige U-förmigen Einbuchtung eine Dimension in der Breite von 5 - 10 cm und in der Länge von 40 - 100 cm aufweisen.

OP-Abdeckungen mit einer Ausnehmung können vorteilhaft für spezielle Operationen sein. So kann durch eine OP-Abdeckung mit einer Ausnehmung der für den chirurgischen Eingriff vorgesehene Bereich des Patienten optimiert abgedeckt werden, wie beispielsweise als eine direkte Begrenzung des Operationsfeldes. Ebenso können in die Ausnehmung der OP-Abdeckung beispielsweise Extremitäten des Patienten, wie Arm oder Bein oder sonstige exponierte Bereiche wie Schulter, Knie aufgenommen und damit umfangen werden.

Insbesondere vorteilhafterweise umgibt der zweite absorbierende Bereich die Ausnehmung, wobei der absorbierende Materialabschnitt den Kanten der Ausnehmung zugeordnete innere Randkanten und innere Randbereiche sowie äußere Randkanten und äußere Randbereiche aufweist.

Insbesondere vorteilhafterweise umgibt der zweite absorbierende Bereich die Ausnehmung unmittelbar und grenzt an diese an.

In einer weiteren vorteilhaften Ausführung umgibt der zweite absorbierende Bereich die Ausnehmung in einem radialen Abstand. Eine radial beabstandete Anordnung des absorbierenden Materialabschnitts in Bezug auf die Kanten der Ausnehmung ist insbesondere der Fall, wenn die eigentliche Ausnehmung primär von einem gummielastischen Material in Form einer Manschette umgeben ist. Bei OP-Abdeckungen, deren Ausnehmungen für die Aufnahme von Extremitäten vorgesehen sind, ist eine gummielastische Manschette für die bessere Passform vorteilhaft.

Der zweite absorbierende Bereich kann die Ausnehmung dabei zumindest abschnittsweise oder insbesondere dann vollumfänglich umgeben.

"Innere Randkanten" bzw. "innere Randbereiche" werden dabei in Bezug auf die Ausnehmung als die proximal angeordneten, also an die Kanten der Ausnehmung unmittelbar angrenzenden Randkanten bzw. Randbereiche des die Ausnehmung umgebenden absorbierenden Materialabschnitts verstanden.

"Äußere Randkanten" bzw. "äußere Randbereiche" werden dabei in Bezug auf die Ausnehmung als die distal angeordneten, also von den Kanten der Ausnehmung entfernt gelegenen Randkanten bzw. Randbereiche des die Ausnehmung umgebenden absorbierenden Materialabschnitts verstanden.

Insbesondere vorteilhaft sind dabei die inneren Randkanten des absorbierenden Materialabschnitts im Wesentlichen bündig zu den Kanten der Ausnehmung angeordnet. Durch diese weitgehend kantenbündige Anordnung des absorbierenden Materialabschnitts kann die aus dem Operationsfeld heraustretende Flüssigkeit durch den optimierten Bereich direkt primär gebunden werden.

"Im Wesentlichen bündig zu den Kanten angeordnet" will so verstanden werden, dass eine minimale produktionstechnisch bedingte Abweichung der Anordnung der inneren Randkanten des absorbierenden Materialabschnitts in Bezug auf die Kanten der Ausnehmung, sei es dass die inneren Randkanten sich über die Kanten der Ausnehmung hinaus erstrecken oder dass die inneren Randkanten innerhalb eines radialen Abstands zu den Kanten der Ausnehmung enden, noch durch den Erfindungsgedanken gestützt wird.

"Produktionstechnisch bedingte Abweichungen" wird als eine Abweichung bis zu höchstens +/ - 5 mm verstanden.

In weiterer Ausführung der OP-Abdeckung ist der absorbierende Materialabschnitt näher an dem oberen Ende der OP-Abdeckung als an dem unteren Ende der OP-Abdeckung angeordnet.
Davon ausgehend, dass das obere Ende das im Gebrauch der OP-Abdeckung dem Operationsfeld nächst gelegene Ende beschreibt, bietet diese Anordnung eine optimierte Flüssigkeitsbindung und auch Flüssigkeitsretardierung. Insbesondere bei einem großen Flüssigkeitsaufkommen kann dadurch die Kontaminationsgefahr aufgrund zu schnellen Abtropfens verringert werden. Entsprechend dem oberen Ende und dem unteren Ende der OP-Abdeckung hat der absorbierende Materialabschnitt ebenso eine "obere Randkante" und einen "oberen Randbereich" sowie eine "untere Randkante" und einen "unteren Randbereich". An die untere Randkante und an den unteren Randbereich schließen sich jeweils seitlich die weiteren Randkanten und die weiteren Randbereiche an.
Die Summe aller Randkanten bildet dann den Umfang des absorbierenden Materialabschnitts.

Besonders vorteilhaft ist diese Ausführungsform der OP-Abdeckung, wenn die obere Randkante des absorbierenden Materialabschnitts im Wesentlichen bündig zum oberen Ende der OP-Abdeckung angeordnet ist.
"Im Wesentlichen bündig zum oberen Ende der OP-Abdeckung", will so verstanden werden, dass eine minimale produktionstechnisch bedingte Abweichung der Anordnung der oberen Randkante des absorbierenden Materialabschnitts in Bezug auf das obere Ende, sei es dass die Randkante des absorbierenden Materialabschnitts sich über das obere Ende der OP-Abdeckung hinaus erstreckt oder dass die Randkante des absorbierenden Materialabschnitts sich minimal bis unterhalb des oberen Endes erstreckt, noch durch den Erfindungsgedanken gestützt wird.

In besonders vorteilhafter Ausführung weist der absorbierende Materialabschnitt an einem unteren Randbereich eine Flüssigkeitsbarriere auf. Hiermit kann die durch die Schwerkraft bedingt nach unten in Richtung Boden wandernde Flüssigkeit zurückgehalten werden.

In der Weiterbildung einer OP-Abdeckung weisen die an einen unteren Randbereich des Materialabschnitts sich jeweils seitlich daran anschließenden Randbereiche des Materialabschnitts ebenso eine Flüssigkeitsbarriere auf.
Durch die seitliche Fortführung der Flüssigkeitsbarriere wird vorteilhaft eine Tasche mit einem dreikantig umrandeten Auslaufschutz gestaltet.

In einer weiteren bevorzugten Ausführungsform weisen nur die äußeren Randbereiche des Materialabschnitts eine Flüssigkeitsbarriere auf.
In einer weiteren bevorzugten Ausführungsform weisen alle Randbereiche des absorbierenden Materialabschnitts eine Flüssigkeitsbarriere auf.

Besonders vorteilhaft erweist sich, wenn unterschiedliche Fügemittel eingesetzt werden derart, dass in den Randbereichen eine erste Flüssigkeitsbarriere und zumindest eine weitere, das heißt von der ersten verschiedene, Flüssigkeitsbarriere gebildet wird.

OP-Abdeckungen werden für ihren definierten Einsatzbereich vom geschulten medizinischen Personal zumeist standardisiert angeordnet, so dass bspw. eine definierte Anordnung in Bezug auf das Operationsfeld gegeben ist.
Aufgrund der Anordnung im bestimmungsgemäßen Gebrauch der OP-Abdeckung kann die Problematik der der Schwerkraft folgenden Flüssigkeitsmigration bereichsweise unterschiedlich stark vertreten sein. Demnach kann es vorteilhaft sein, unterschiedliche Fügemittel einzusetzen und damit unterschiedliche Formen an Flüssigkeitsbarrieren bereitzustellen.

Die durch die Fügemittel bereitgestellte Flüssigkeitsbarriere kann dabei auf der Innenseite und/oder der Außenseite des absorbierenden Materialabschnitts angeordnet sein.
Die Anordnung der Flüssigkeitsbarriere ergibt sich aus der Anordnung und der Fixierung des jeweiligen Fügemittels.

Für die Ausbildung von Flüssigkeitsbarrieren in den Randbereichen sind unterschiedliche Anordnungen des Fügemittels vorstellbar.

In einer ersten bevorzugten Ausführungsform ist das Fügemittel planar zwischen der Oberseite der Basisabdeckmaterials und der Innenseite des absorbierenden Materialabschnitts angeordnet.

In einer zweiten bevorzugten Ausführungsform ist das Fügemittel planar zwischen der Oberseite der Basisabdeckmaterials und der Innenseite des absorbierenden Materialabschnitts angeordnet, wobei die durch das Fügemittel gebildete Flüssigkeitsbarriere sich über die Breite des fixierten Randbereiches in Richtung Tasche hinaus erstreckt. D.h. insbesondere ist das Fügemittel zwischen der Oberseite des Basisabdeckmaterials und der Innenseite des absorbierenden Materialabschnitts und diese direkt verbindend angeordnet und erstreckt sich weiter in Richtung Tasche hinaus, wobei das Fügemittel dann nur noch auf der Innenseite des absorbierenden Materialabschnitts angeordnet ist.

In einer dritten bevorzugten Ausführungsform ist das Fügemittel zwischen der Oberseite des Basisabdeckmaterials und der Innenseite des absorbierenden Materialabschnitts und diese direkt verbindend angeordnet und erstreckt sich weiter über die Randkante des absorbierenden Materialabschnittes hinaus und ist dann zurückgefaltet auf der Oberseite des absorbierenden Materialabschnittes fixiert. Diese bevorzugte Ausformungsform des Fügemittels ist insbesondere vorteilhaft, da der absorbierende Materialabschnitt zusätzlich auch an der Randkante und diese von außen durch die mittels des Fügemittels geschaffene Flüssigkeitsbarriere abgedichtet ist.

In einer vierten bevorzugten Ausführungsform ist das Fügemittel an der Oberseite des Basisabdeckmaterials angeordnet und erstreckt sich weiter über die Randkante des absorbierenden Materialabschnittes hinaus und ist dann zurückgefaltet auf der Oberseite des absorbierenden Materialabschnittes fixiert. Diese bevorzugte Ausformungsform des Fügemittels ist ebenso vorteilhaft, da der absorbierende Materialabschnitt zusätzlich auch an der Randkante und diese von außen durch die mittels des Fügemittels geschaffene Flüssigkeitsbarriere abgedichtet ist.

In einer fünften bevorzugten Ausführungsform ist das Fügemittel rinnenförmig konfiguriert und die beiden Schenkel der Rinne sind ausgehend von der distal angeordneten Faltkante jeweils auf der Oberseite des Basisabdeckmaterials und auf der Innenseite des absorbierenden Materialabschnitts fixiert, so dass sich die Rinne in Richtung auf die Tasche öffnet. Die Faltkante kann dabei vorzugsweise mittig angeordnet sein. Die Faltkante kann in Bezug auf die Gesamtbreite des Fügemittels auch exzentrisch angeordnet sein.

In einer sechsten bevorzugten Ausführungsform überfängt das Fügemittel sowohl einen Randbereich auf der Außenseite des absorbierenden Materialabschnitts als auch einen daran unmittelbar angrenzenden Teil der Oberseite des Basisabdeckmaterials.

In einer siebten bevorzugten Ausführungsform ist das Fügemittel in Form von Schweißstellen ausgebildet. Die Schweißstellen können dabei vorzugsweise Ultraschallschweißstellen, thermische Schweißstellen (thermobonding) oder Kalanderschweißstellen sein. Die Schweißstellen sind dabei vorzugsweise in Form eines Schweißmusters angeordnet. Das Schweißmuster kann sich dabei aus der Anordnung der Schweißstellen und deren Abstand zueinander, also der Schweißstellendichte ergeben. Ebenso kann das Schweißmuster aus der unterschiedlichen Tiefe der Schweißstellen resultieren.
Das Fügemittel in Form eines Schweißmusters erstreckt sich zumindest über den Randbereich. Vorzugsweise erstreckt sich das Fügemittel auch noch über die Randkante und den daran angrenzenden Teil des Basisabdeckmaterials.
Besonders vorteilhaft ist das Schweißmuster derart gestaltet, dass es in der Richtung von Randkante in Richtung Tasche ein abnehmendes Profil hinsichtlich Bindestellentiefe und/oder Bindestellendichte und/oder Bindestellenstärke aufweist. Die Bindungsstärke der jeweiligen Schweißstellen kann durch die Wahl der Ultraschallstärke bzw. der Temperatur bzw. des Druckes eingestellt werden.

Besonders vorteilhaft weist das Schweißmuster einen ersten Teilbereich, welcher proximal zur Randkante angeordnet ist, mit einem ersten Schweißteilmuster auf und einen weiteren, in Richtung Tasche weisenden zweiten Teilbereich mit einem zweiten Schweißteilmuster. Insbesondere vorteilhaft sind das erste und zweite Schweißteilmuster durch eine Schweißvorrichtung mit einem Temperaturprofil gebildet.

Insbesondere weist das erste Schweißteilmuster Schweißstellen für die unlösbare Fixierung des absorbierenden Materialabschnitts auf dem Basisabdeckmaterial auf und das zweite Schweißteilmuster weist nur Schweißstellen auf, welche nur auf dem absorbierenden Materialabschnitt angeordnet sind.

Die Erfindung erweist sich auch als besonders vorteilhaft, wenn die Flüssigkeitsbarriere sich über die Breite des fixierten Randbereiches in Richtung Tasche hinaus erstreckt.

Außerdem erweist es sich insbesondere als vorteilhaft, wenn die unlösbar fixierten Randbereiche eine Fläche von 2 - 45 % , insbesondere von 2 - 35 %, weiter insbesondere von 2- 30 %, bezogen auf die Gesamtfläche des absorbierenden Materialabschnitts einnehmen.

Des Weiteren erweist es sich als vorteilhaft, wenn die fixierten Randbereiche eine Breite B von 0,5 - 10 cm, vorzugsweise von 1 - 8 cm, weiter vorzugsweise von 1,5 - 6 cm aufweisen.

Insbesondere vorteilhaft ist die Breite in den fixierten Randbereichen unterschiedlich. Insbesondere vorteilhaft weist der untere Randbereich im Vergleich zu den seitlich an den unteren Rand anschließenden weiteren Randbereichen und/oder im Vergleich zum oberen Randbereich eine größere Breite B auf.

Beim bestimmungsgemäßen Gebrauch einer OP-Abdeckung sollte die während eines chirurgischen Eingriffs freigesetzte und aus dem Operationsfeld heraustretende Flüssigkeit optimal gebunden werden.
Durch den zweiten absorbierenden Bereich wird hiermit ein für diesen kritischen Moment optimierter Bereich innerhalb der OP-Abdeckung zur Verfügung gestellt.
Es erweist dabei sich dabei besonders vorteilhaft, wenn der den zweiten absorbierenden Bereich überfangende absorbierende Materialabschnitt eine größere Absorptionskapazität AK aufweist als das den ersten absorbierenden Bereich bildende Material.
Es hat weiterhin als vorteilhaft erwiesen, wenn der absorbierende Materialabschnitt eine um mindestens 25% größere, insbesondere eine um mindestens 50% größere, insbesondere eine um mindestens 100% größere, insbesondere eine um mindestens 150% größere, weiter insbesondere eine um mindestens 200% größere, weiter insbesondere eine um mindestens 250% größere, weiter insbesondere eine um mindestens 300% größere Absorptionskapazität AK aufweist als das den ersten Bereich bildende Material.

Zum Erhalt der Absorptionskapazität AK (ausgedrückt in g/m²) wird das jeweilige Material nach der Methode EN ISO 9073-6:2003 vermessen. Das nach dieser Methode vermessene Aufnahmevermögen (ausgedrückt als g/g, also Gewicht Flüssigkeit bezogen auf das Gewicht des Materials) wird dann auf das Flächengewicht des Materials bezogen, unter Erhalt der Einheit g/m².

Vorteilhafterweise weist das den absorbierenden Materialabschnitt bildende Material eine Absorptionskapazität AK von mindestens 150 g/m², insbesondere mindestens 200 g/m², insbesondere mindestens 250 g/m², insbesondere mindestens 300 g/m², insbesondere mindestens 350 g/m² auf, mit der Absorptionskapazität AK gemessen und berechnet nach der voranstehend beschriebenen Methode.

Weiter vorteilhafterweise weist das den ersten Bereich bildende Material eine Absorptionskapazität AK von 50 - 250 g/m², insbesondere 80 - 220 g/m², weiter insbesondere von 100 - 200 g/m² auf, mit der Absorptionskapazität AK gemessen und berechnet nach der voranstehend beschriebenen Methode.

Es hat sich auch als vorteilhaft erwiesen, wenn der zweite absorbierende Bereich eine flächenhafte Erstreckung von 2 - 65 %, vorzugsweise 2 - 55 %, weiter vorzugsweise 2 - 45 % bezogen auf die Gesamtfläche des Basisabdeckmaterials aufweist.
Durch diesen Flächenanteil des zweiten absorbierenden Bereichs kann die OP-Abdeckung insgesamt ein dennoch optimiertes Flüssigkeitsmanagement bereitstellen, verbunden mit Materialeinsparung und damit Kosteneinsparung.

Das Flächengewicht des absorbierenden Materialabschnitts beträgt vorzugsweise 25 - 100g/m², insbesondere 35 - 80 g/m², weiter insbesondere 45 - 65 g/m², weiter vorzugsweise von 50 - 60 g/m².

In besonders vorteilhafter Weise ist der absorbierende Materialabschnitt durch einen ein-oder mehrlagigen Vliesstoff gebildet. Besonders bevorzugt ist der absorbierende Materialabschnitt durch einen hydrophilisierten Vliesstoff mit ein oder mehreren Lagen aus Spunbond (S) und/oder Meltblown (M) gebildet. Insbesondere bevorzugt ist der absorbierende Materialabschnitt durch ein hydrophilisertes SMS-Vlies aus Polypropylen gebildet.

In besonders vorteilhafter Weise ist das Basisabdeckmaterial aus einem ein- oder mehrlagigen Vliesstoff, insbesondere einem Laminat aus einer oder mehreren Spinnvlies- und/oder Meltblownvlieslagen oder einem Vlies-Folien-Verbund gebildet.

Besonders bevorzugt ist das Basisabdeckmaterial durch einen Vlies-Folien-Verbund mit zur Unterseite gerichteten Folie gebildet.

Im Falle eines Vlies-Folien-Verbundes beträgt das Flächengewicht des Vlieses vorzugsweise 10 - 40 g/m², insbesondere 10-35 g/m², weiter insbesondere 15-35 g/m², weiter insbesondere 20-35 g/m². Die Folie hat ein Flächengewicht von vorzugsweise 15 - 35 g/m², insbesondere 20 - 30 g/m².

Insbesondere bevorzugt ist das Basisabdeckmaterial durch einen Vlies-Folien-Verbund mit einem hydrophilisierten Vliesstoff mit ein oder mehreren Lagen aus Spunbond und/oder Meltblown gebildet.

Die Folie im Vlies-Folien-Verbund ist vorzugsweise eine nicht mikroporöse Folie.

Die Laminierung des Vlies-Folien-Verbundes kann auf jegliche Art erfolgen, vorzugweise sind Vlies-Folie mittels Thermobonding und/oder Hotmelt verbunden.
Insbesondere bevorzugt ist das Basisabdeckmaterial durch einen Verbund aus hydrophilisertem Spunbond-Vlies aus Polypropylen mit einer PE-Folie gebildet. In einer weiteren vorteilhaften Weiterbildung der OP-Abdeckung weist die Oberseite weitere zweite absorbierende Bereiche auf.
Dieser eine und der weitere zweite absorbierende Bereich können unmittelbar zueinander angrenzend oder mit einem Abstand voneinander auf der Oberseite angeordnet sein.
Die zweiten absorbierenden Bereiche können sich vorzugsweise in ihrer jeweiligen flächenhaften Erstreckung unterscheiden. Weiter vorzugsweise sind die zweiten absorbierenden Bereiche von absorbierenden Materialabschnitten überfangen, welche sich unterscheiden, so beispielsweise in ihrer Absorptionskapazität, in ihrem Flächengewicht, in ihrer Materialzusammensetzung.
Vorzugsweise weist sowohl der eine zweite absorbierende Bereich als auch der weitere zweite absorbierende Bereich eine Ausnehmung auf.

Besonders vorteilhafte Ausführungen der erfindungsgemäßen OP-Abdeckungen :
Eine besonders bevorzugte Ausführung der OP-Abdeckung umfasst ein Basisabdeckmaterial besteht aus einem Vlies-Folien-Verbund, wobei die zur Oberseite gerichtete Schicht des Vlieses aus einem hydrophilisiertem Spinnvlies, insbesondere aus einem hydrophilisiertem PP-Spinnvlies besteht. Das Flächengewicht der Spinnvlies-Lage beträgt 20 - 35 g/m². Die Absorptionskapazität dieses Spinnvlies beträgt nach der oben beschriebenen Methode 120 - 180 g/m².

Der den zweiten absorbierenden Bereich überfangende absorbierende Materialabschnitt ist ist aus einem hydrophilisiertem SMS-Vlies, insbesondere aus einem hydrophilisiertem PP-SMS-Vlies mit einem Flächengewicht von 50 - 60 g/m² gebildet. Die Absorptionskapazität dieses SMS-Vlieses beträgt nach der oben beschriebenen Methode 220 - 330 g/m².
Die OP-Abdeckung erstreckt sich in Längsrichtung LR zwischen einem oberen Ende und einem unteren Ende, wobei der absorbierende Materialabschnitt näher am oberen Ende der OP-Abdeckung als an dem unteren Ende der OP-Abdeckung angeordnet. In dieser Anordnung weist auch der Materialabschnitt entsprechend eine obere, eine untere und davon anschließende seitliche Randkanten und entsprechende an diese Randkanten angrenzende Randbereiche auf. Der Materialabschnitt ist insbesondere vorteilhaft mit seiner oberen Randkante im Wesentlichen bündig zum oberen Ende der OP-Abdeckung angeordnet.
Der obere Randbereich, der untere Randbereich und die davon anschließenden seitlichen Randbereiche weisen eine Flüssigkeitsbarriere auf. Die Flüssigkeitsbarriere wird durch Fügemittel gebildet, welche den Materialabschnitt unlösbar auf der Oberseite des Basisabdeckmaterials fixieren.
Dabei wird für den unteren Randbereich ein von den weiteren Randbereichen (also den an den unteren Rand seitlich anschließenden Randbereichen und dem oberen Randbereich) unterschiedliches Fügemittel eingesetzt, so dass der untere Randbereich eine erste Flüssigkeitsbarriere und die weiteren Randbereiche eine weitere, das heißt von der ersten verschiedene, Flüssigkeitsbarriere aufweisen.

Vorzugsweise werden in der vorangehend beschriebenen OP-Abdeckung folgende Kombinationen an Fügemitteln eingesetzt.

In einer ersten bevorzugten Variante ist das Fügemittel im unteren Randbereich rinnenförmig konfiguriert, entsprechend der oben beschriebenen fünften Ausführungsform des Fügemittels.

In einer zweiten bevorzugten Variante ist das Fügemittel im unteren Randbereich entsprechend der vorangehend näher erläuterten vierten Ausführungsform des Fügemittels gestaltet und fixiert.
In einer dritten bevorzugten Variante ist das Fügemittel im unteren Randbereich entsprechend der vorangehend näher erläuterten sechsten Ausführungsform des Fügemittels gestaltet und fixiert.

In einer vierten bevorzugten Variante ist das Fügemittel im unteren Randbereich entsprechend der vorangehend näher erläuterten siebten Ausführungsform des Fügemittels gestaltet und fixiert.

In einer fünften bevorzugten Variante ist das Fügemittel im unteren Randbereich entsprechend der vorangehend näher erläuterten zweiten Ausführungsform des Fügemittels gestaltet und fixiert.

In einer sechsten bevorzugten Variante ist das Fügemittel im unteren Randbereich entsprechend der vorangehend näher erläuterten dritten Ausführungsform des Fügemittels gestaltet und fixiert.

Für alle genannten Varianten werden in den weiteren Randbereichen vorzugsweise Fügemittel eingesetzt, welche entsprechend der näher erläuterten ersten Ausführungsform oder auch vorzugsweise entsprechend der näher erläuterten zweiten bevorzugten Ausführungsform des Fügemittels planar zwischen der Oberseite des Basisabdeckmaterials und der Innenseite des absorbierenden Materialabschnitts angeordnet sind. Insbesondere sind diese Randbereiche mittels eines Hotmeltklebers fixiert. Weiter insbesondere sind diese Randbereiche mittels eines doppelseitigen nässestabilen Klebebands fixiert.

In weiterer vorteilhafter Ausbildung der OP-Abdeckung weisen der untere Randbereich und auch die jeweils seitlich daran anschließenden Randbereiche eine Flüssigkeitsbarriere auf. Vorteilhafterweise werden die voranstehend beschriebene Varianten an Fügemitteln im unteren Randbereich auch in den seitlich daran anschließenden Randbereichen weitergeführt, so dass nur der obere Randbereich vorzugsweise mit einem planar zwischen der Oberseite des Basisabdeckmaterials und der Innenseite des absorbierenden Materialabschnitts, also insbesondere in Form eines Hotmeltklebers oder auch eines zweiseitigen Klebebandes unter Bildung der weiteren Flüssigkeitsbarriere fixiert ist.

In weiterer vorteilhafter Ausbildung der OP-Abdeckung weisen alle Randbereiche, also der obere und der untere und auch die an den unteren seitlich anschließenden Randbereiche eine Flüssigkeitsbarriere auf.
Weiter vorteilhafterweise wird die Flüssigkeitsbarriere durch das gleiche Fügemittel gebildet.

In einer weiteren besonders bevorzugten Ausführungsform weist die vorstehend beschriebene OP-Abdeckung eine Ausnehmung auf.
Die an ihren jeweiligen äußeren und inneren Randbereichen eingesetzten Fügemittel unterscheiden sich, so dass entsprechend im äußeren und im inneren Randbereich zumindest abschnittsweise eine erste und eine weitere, das heißt von der ersten verschiedene, Flüssigkeitsbarriere gebildet wird.
In einer insbesonderen bevorzugten Weiterbildung sind in den äußeren und inneren Randbereichen, und wiederum in diesen ihrerseits vorhandenen oberen, unteren und in den an den unteren Randbereich seitlichen anschließenden Randbereichen unterschiedliche Fügemittel vorhanden

Weitere Merkmale, Vorteile und Einzelheiten der Erfindung ergeben sich aus den beigefügten Patentansprüchen und aus der zeichnerischen Darstellung und nachfolgenden Beschreibung bevorzugter Ausführungsformen der Erfindung.
Für die vorstehend offenbarten Merkmale und die nachfolgend in den Patentansprüchen zum Ausdruck gebrachten Merkmale wird unabhängig einer etwaigen Rückbeziehung und in beliebiger Kombination miteinander Patentschutz in Anspruch genommen.

Die Erfindung soll im Folgenden anhand von Figuren näher erläutert werden, dabei zeigen
- Figur 1:: schematisch eine Draufsicht einer erfindungsgemäßen OP-Abdeckung
- Figur 2:: schematisch eine Seitenansicht der OP-Abdeckung aus Figur 1 entlang der Linie Q ---- Q
- Figur 3:: schematisch eine Draufsicht auf eine weitere erfindungsgemäße OP-Abdeckung mit einer Ausnehmung
- Figur 4:: schematisch eine Draufsicht auf eine weitere erfindungsgemäße OP-Abdeckung mit einer Ausnehmung
- Figur 5:: schematisch eine Draufsicht auf eine OP-Abdeckung nach Figur 1 mit Anordnung der Flüssigkeitsbarrieren in einer bevorzugter Ausführungsform
- Figur 6:: schematisch eine Draufsicht auf eine OP-Abdeckung nach Figur 1 mit Anordnung der Flüssigkeitsbarrieren in einer weiteren bevorzugten Ausführungsform
- Figur 7:: schematisch eine Draufsicht auf eine OP-Abdeckung nach Figur 3 mit Anordnung der Flüssigkeitsbarrieren in einer bevorzugten Ausführungsform
- Figuren 8 - 13:: jeweils schematisch das Fügemittel in bevorzugter Ausführungsform, angeordnet in der OP-Abdeckung aus Figur 1 entlang der Linie Q --- Q
- Figur 14:: schematisch eine Draufsicht auf eine OP-Abdeckung mit einem Fügemittel in einer weiteren bevorzugten Ausführungsform
- Figur 15:: schematisch das Fügemittel, angeordnet in der OP-Abdeckung aus Figur 14 entlang der Linie Q --- Q
- Figur 16:: schematisch eine Draufsicht auf eine OP-Abdeckung mit einem Fügemittel in Form eines Schweißmusters
- Figur 17:: schematisch das Fügemittel, angeordnet in der OP-Abdeckung aus Figur 16, entlang der Linie Q --- Q
- Figur 18:: schematisch die Fügemittel, angeordnet in der OP-Abdeckung aus Figur 7, entlang der Linie Q --- Q
- Figur 19:: schematisch die Anwendung der OP-Abdeckung nach Figur 1
- Figur 20:: schematisch die Anwendung der OP-Abdeckung nach Figur 3

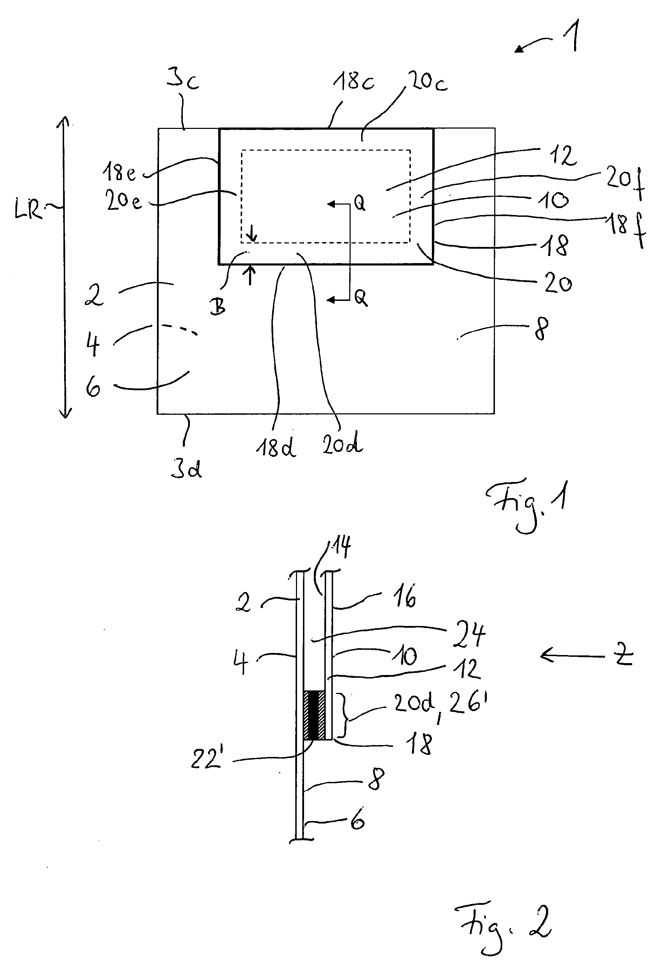

Figur 1 zeigt schematisch die Draufsicht auf eine OP-Abdeckung 1 in bevorzugter rechteckförmiger Ausführungsform. Die OP-Abdeckung 1 weist dabei in einer Längsrichtung LR ein oberes Ende 3c und ein unteres Ende 3d auf. Als Längsrichtung LR wird dabei jegliche flächenhafte Erstreckung zwischen zwei sich gegenüber liegenden Enden des Flächengebildes verstanden. Das obere Ende 3c und das untere Ende 3d der OP-Abdeckung 1 wird durch die im Gebrauch der OP-Abdeckung vorgesehene Anordnung in Relation zur Bodenebene definiert.
Die OP-Abdeckung 1 hat dabei ein Basisabdeckmaterial 2, welches
flüssigkeitsundurchlässig ist. Das Basisabdeckmaterial 2 hat dabei eine in Gebrauch zum Patienten gerichtete Unterseite 4 und eine Oberseite 6. Diese Oberseite 6 weist dabei eine absorbierende Schicht auf. Zudem hat die Oberseite 6 einen ersten absorbierenden Bereich 8 und einen zweiten absorbierenden Bereich 10.
Dieser zweite absorbierende Bereich 10 ist dabei von einem Materialabschnitt 12 überfangen. Der absorbierende Materialabschnitt 12 hat dabei Randkanten 18 und an die Randkanten 18 angrenzende Randbereiche 20. Entsprechend dem oberen Ende 3c und dem unteren Ende 3c der OP-Abdeckung 1 hat der absorbierende Materialabschnitt 12 ebenso eine obere Randkante 18c und einen oberen Randbereich 20c sowie eine untere Randkante 18d und einen unteren Randbereich 20d. An die untere Randkante 18d und an den unteren Randbereich 20d schließen sich jeweils seitlich die weiteren Randkanten 18e, 18f und die weiteren Randbereiche 20e, 20f an.
Die Summe aller Randkanten 18c, 18d, 18e, 18f bildet dann den Umfang des absorbierenden Materialabschnitts 12.

Der zweite absorbierende Bereich 10 wird durch die flächenhafte Erstreckung des absorbierenden Materialabschnitts 12 begrenzt. Der zweite absorbierende Bereich 10 enthält den absorbierenden Materialabschnitt 12 und auch das in Z-Richtung projizierte (wie in Figur 2 angedeutet), mit derselben flächenhaften Erstreckung begrenzt darunter liegende Basisabdeckmaterial 2.

Diejenige, verbleibende Fläche der Oberseite 6 des Basisabdeckmaterials 2, die nicht durch einen absorbierenden Materialabschnitt 12 abgedeckt ist, bildet den ersten absorbierenden Bereich 6. Die Randbereiche 20 weisen eine Breite B von 1,5 - 6 cm auf.

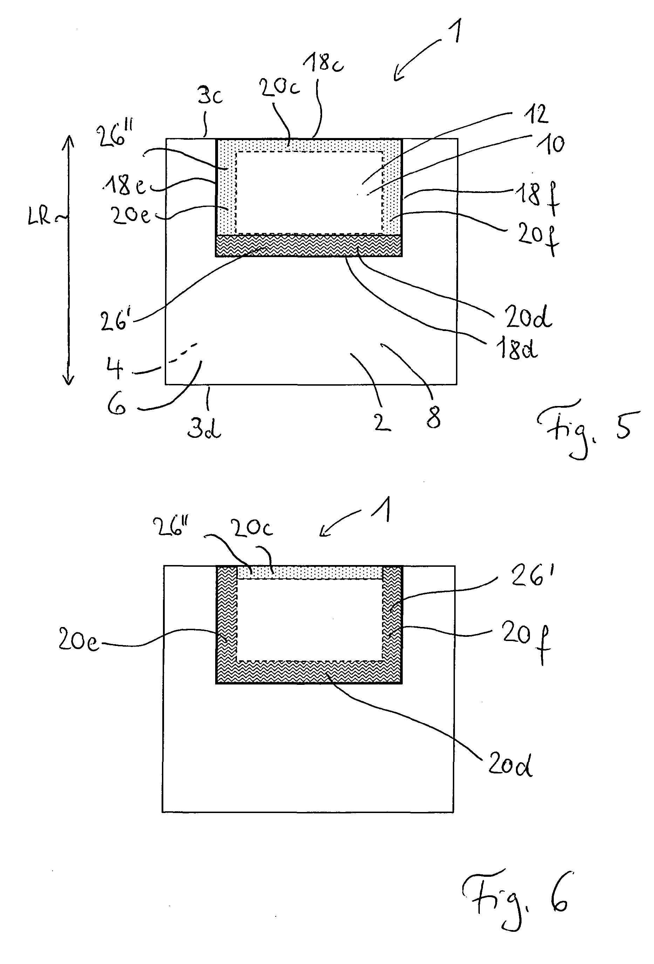

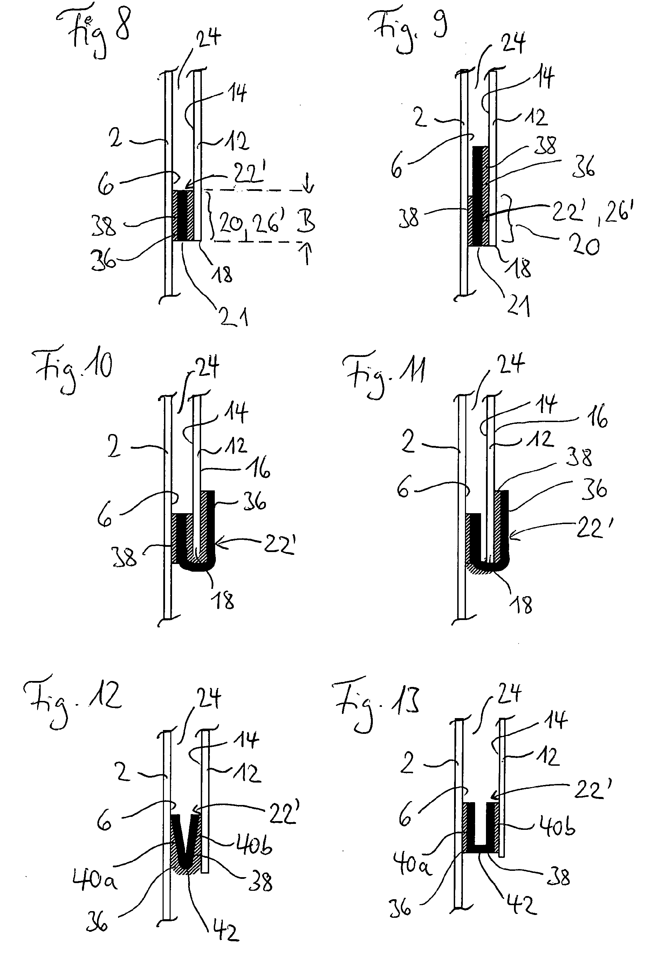

Figur 2 zeigt eine Seitenansicht der OP-Abdeckung 1 entlang der Linie Q---Q aus Figur 1. Der Ausschnitt ist nur beispielhaft aus dem unteren Randbereich des absorbierenden Materialabschnitts ausgewählt. Die Anordnung wie nachfolgend beschrieben kann an allen Randbereichen vorliegen.
Der absorbierende Materialabschnitt 12 ist in seinen Randbereichen 20c, 20d, 20e, 20f vollumfänglich auf der Oberseite 6 des Basisabdeckmaterials 2 unlösbar fixiert. Weiterhin ist der absorbierende Materialabschnitt 12 außerhalb dieser Randbereiche 20c, 20d, 20e, 20f im Wesentlichen nicht mit dem Basisabdeckmaterial 2 verbunden, wobei darunter verstanden wird, dass weitere Bindungen des absorbierenden Materialabschnitts mit dem Basisabdeckmaterial außerhalb der bereits fixierten Randbereiche höchstens in einem Anteil von 10% bezogen auf die Gesamtfläche des absorbierenden Materialabschnitts vorhanden sind.
Die Fixierung ist damit derart, dass zwischen dem absorbierenden Materialabschnitt 12 und dem flüssigkeitsundurchlässigen Basisabdeckmaterial 2 eine Tasche 24 gebildet ist, welche vollumfänglich verschlossen ist.
Die Randbereiche 20c, 20d, 20e, 20f des absorbierenden Materialabschnittes 12 weisen zumindest abschnittweise eine Flüssigkeitsbarriere 26' auf, wobei diese Flüssigkeitsbarriere 26' durch die unlösbare Fixierung des absorbierenden Materialabschnitts in seinen Randbereichen 20c, 20d, 20e, 20f auf der Oberseite 6 des Basisabdeckmaterials 2 durch das Fügemittel 22' gebildet ist.
Die Flüssigkeitsbarriere 26' wird zumindest in dem unterem Randbereich 20d gebildet, so wie schematisch dann in Figur 5 dargestellt ist.
Die Flüssigkeitsbarriere 26' kann auch zusätzlich in den an den unteren Randbereich 20d seitlich anschließenden Randbereichen 20e und 20f gebildet sein, so wie schematisch in Figur 6 dargestellt ist.
Das Fügemittel 22' kann dabei unterschiedliche bevorzugte Ausführungsformen, wie nachfolgend noch in den Figuren 8 - 18 detaillierter beschrieben wird, einnehmen.

Der absorbierende Materialabschnitt 12 ist dabei näher an dem oberen Ende 3c der OP-Abdeckung 1 als an dem unteren Ende 3d der OP-Abdeckung 1 angeordnet. Der absorbierende Materialabschnitt 12 ist dabei sogar derart angeordnet, dass die obere Randkante 18c im Wesentlichen bündig zum oberen Ende 3c der OP-Abdeckung 1 abschließt.
Im Wesentlichen bündig zum oberen Ende der OP-Abdeckung, will so verstanden werden, dass minimale produktionstechnisch bedingte Abweichungen der Anordnung der oberen Randkante 18c des absorbierenden Materialabschnitts 12 in Bezug auf das obere Ende 3c, sei es dass die Randkante des absorbierenden Materialabschnitts sich über das obere Ende 3c der OP-Abdeckung hinauserstreckt oder dass die Randkante 18c des absorbierenden Materialabschnitts sich minimal bis unterhalb des oberen Endes 3c erstreckt, noch durch den Erfindungsgedanken gestützt wird.

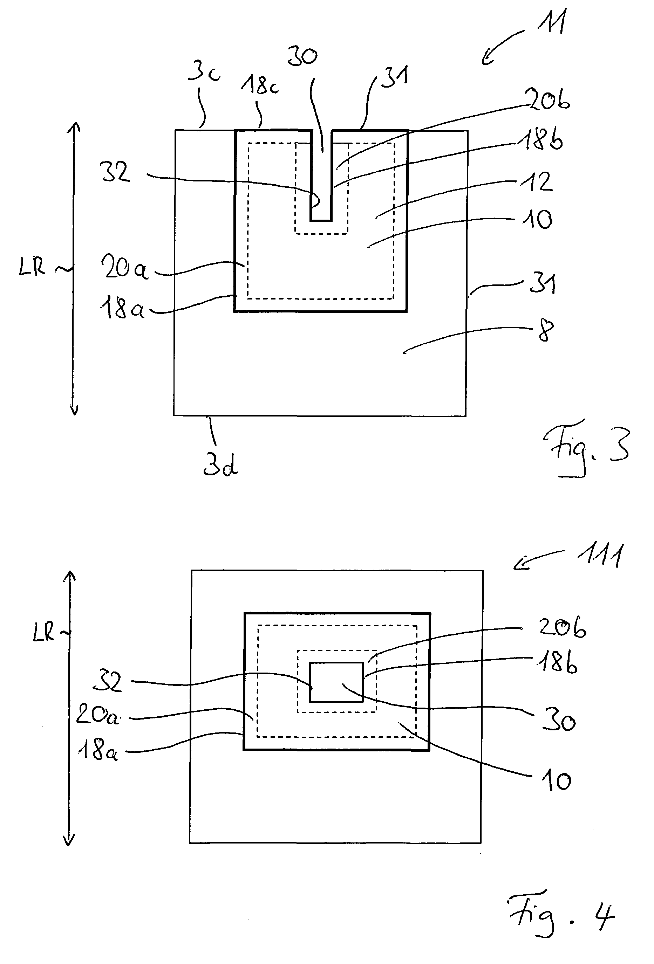

Die Figuren 3 und 4 zeigen jeweils schematisch eine Draufsicht auf weitere bevorzugte Ausführungsformen der OP-Abdeckung.
Figur 3 zeigt eine OP-Abdeckung 11 mit einer Ausnehmung 30. Dabei wird ausgehend von einer primär wahrgenommenen äußeren Kontur, gebildet durch die Gesamtheit aller äußeren Kanten 31, eine Einbuchtung in Richtung Mitte der OP-Abdeckung geführt, welche dann die Ausnehmung 30 darstellt. Die Ausnehmung 30 wird durch ihre Kanten 32, in diesem Fall nur unvollständig, begrenzt.
In Analogie zur bereits voranstehend beschriebenen OP-Abdeckung 1 ist auch bei dieser Ausführungsform der OP-Abdeckung 11 ein absorbierender und flüssigkeitsdurchlässiger Materialabschnitt 12 in seinen Randbereichen vollumfänglich auf der absorbierenden Oberseite des flüssigkeitsundurchlässigen Basisabdeckmaterials unlösbar und unter Ausbildung einer Tasche fixiert.
Der zweite absorbierende Bereich 10 umgibt die Ausnehmung 30 unmittelbar. Der absorbierende Materialabschnitt weist in Bezug auf die Kanten 32 der Ausnehmung 30 innere Randkanten 18b und innere Randbereiche 20b sowie äußere Randkanten 18a und äußere Randbereiche 20a auf. Der Umfang des absorbierenden Materialabschnitts 12 wird demnach durch die Summe der inneren und äußeren Randkanten 18b, 18a gebildet.
Die inneren Randkanten 18b des absorbierenden Materialabschnitts 12 sind im Wesentlichen bündig zu den Kanten 32 der Ausnehmung 30 angeordnet.
Im Wesentlichen bündig zu den Kanten angeordnet will so verstanden werden, dass eine minimale produktionstechnisch bedingte Abweichung der Anordnung der inneren Randkanten 18b des absorbierenden Materialabschnitts 12 in Bezug auf die Kanten 32 der Ausnehmung 30, sei es dass die inneren Randkanten sich über die Kanten der Ausnehmung hinaus erstrecken oder dass die inneren Randkanten innerhalb eines radialen Abstands zu den Kanten der Ausnehmung enden, noch durch den Erfindungsgedanken gestützt wird.

Der absorbierende Materialabschnitt 12 ist dabei näher an dem oberen Ende 3c der OP-Abdeckung 11 als an dem unteren Ende 3d der OP-Abdeckung 11 angeordnet. Der absorbierende Materialabschnitt 12 ist dabei sogar derart angeordnet, dass die obere Randkante 18c, welche zudem auch eine äußere Randkante 18a darstellt, im Wesentlichen bündig zum oberen Ende 3c der OP-Abdeckung 11 angeordnet ist, wobei minimale produktionstechnisch bedingte Abweichungen von der kantenbündigen Anordnung noch als innerhalb der Erfindung liegend verstanden werden.

Figur 4 zeigt eine OP-Abdeckung 111 mit einer Ausnehmung 30, welche vollumfänglich vom zweiten absorbierenden Bereich 10 umgeben ist. Der absorbierende Materialabschnitt 12 weist in Bezug auf die Kanten 32 der Ausnehmung ebenso innere Randkanten 18b und innere Randbereiche 20b sowie äußere Randkanten 18a und äußere Randbereiche 20a auf. Die inneren Randkanten 18b des absorbierenden Materialabschnitts 12 sind im Wesentlichen bündig zu den Kanten 32 der Ausnehmung 30 angeordnet, wobei minimale produktionstechnisch bedingte Abweichungen von der kantenbündigen Anordnung noch als innerhalb der Erfindung liegend verstanden werden.
Auch hierbei ist der absorbierende Materialabschnitt 12 in seinen Randbereichen vollumfänglich an die Oberseite des Basisabdeckmaterials fixiert. Zumindest einer der Ränder weist zumindest abschnittsweise eine Flüssigkeitsbarriere auf, welche durch Fügemittel bei der unlösbaren Fixierung von absorbierendem Material auf der Oberseite des Basisabdeckmaterials gebildet ist.

Das Fügemittel kann dabei unterschiedliche bevorzugte Ausführungsformen, wie nachfolgend noch in den Figuren 8 - 18 detaillierter beschrieben wird, einnehmen.

Die Figuren 5 und 6 zeigen schematisch eine Draufsicht auf eine, wie in Figur 1 beschriebene OP-Abdeckung 1 mit der bevorzugten Ausführung, dass ein unterer Randbereich 20d des absorbierenden Materialabschnitts 12 eine Flüssigkeitsbarriere 26' aufweist. Die Flüssigkeitsbarriere 26' wird durch die unlösbare Fixierung eines Fügemittels an die Oberseite 6 des Basisabdeckmaterials 2 gebildet. Das Fügemittel kann dabei unterschiedliche bevorzugte Ausführungsformen, wie nachfolgend noch in den Figuren 8 - 17 detaillierter beschrieben wird, einnehmen.
Der absorbierende Materialabschnitt 12 ist in seinen übrigen Randbereichen 20c, 20e, 20f zwar ebenso auf die Oberseite 6 des Basisabdeckmaterials 2 fixiert, jedoch sind in diesen Randbereichen 20c, 20e, 20f keine Flüssigkeitsbarrieren oder in einer weiteren bevorzugten Ausführungsform weitere Flüssigkeitsbarrieren 26" (siehe nachfolgend) durch die Fügemittel ausgebildet.

Figur 6 skizziert schematisch eine Flüssigkeitsbarriere 26', welche zusätzlich auch in den an den unteren Randbereich 20d seitlich anschließenden Randbereichen 20e und 20f gebildet ist. Der absorbierende Materialabschnitt 12 ist in seinem oberen Randbereich 20c zwar ebenso auf die Oberseite 6 des Basisabdeckmaterials2 fixiert, jedoch ist in diesem Randbereich 20c keine Flüssigkeitsbarriere oder sind in einer weiteren bevorzugten Ausführungsform weitere Flüssigkeitsbarrieren 26" (siehe nachfolgend) durch das Fügemittel ausgebildet.

Mittels den Figuren 5 und 6 soll ebenso skizziert werden, dass die Flüssigkeitsbarrieren 26', 26" sich unterscheiden können, und zwar im dem Sinne, dass in den Randbereichen 20c, 20d, 20e, 20f des absorbierenden Materialabschnitts 12 unterschiedliche Fügemittel eingesetzt werden.
So zeigt Figur 5 eine OP-Abdeckung 1 mit einem absorbierenden Materialabschnitt 12, dessen unterer Randbereich 20d eine erste Flüssigkeitsbarriere 26' aufweist, welche von der weiteren Flüssigkeitsbarriere 26" in den daran anschließenden Randbereichen 20e, 20f und dem gegenüberliegenden oberen Randbereich 20c verschieden ist. Die Unterschiede in den Flüssigkeitsbarrieren 26', 26" kann durch die Wahl der Fügemittel erreicht werden.
Der untere Randbereich 20d weist eine erste Flüssigkeitsbarriere 26' auf, welche bessere Barriereeigenschaften innehat als die weitere Flüssigkeitsbarriere 26" in den übrigen Randbereichen 20d, 20e, 20f.

So zeigt die Figur 6 eine OP-Abdeckung 1 mit einem absorbierenden Materialabschnitt 12, dessen oberer Randbereich 20c eine im Vergleich zu den übrigen Randbereichen 20d, 20e, 20f weitere Flüssigkeitsbarriere 26" aufweist. Damit weist die OP-Abdeckung eine integrale Tasche mit einem optimierten Auslaufschutz entlang von drei Randbereichen 20d, 20e, 20f auf.

Die Flüssigkeitsbarriere 26', 26" kann aber auch in allen Randbereichen 20c, 20d, 20e, 20f des absorbierenden Materialabschnitts 12 gebildet sein.
So kann in allen Randbereichen 20c, 20d, 20e, 20f dasselbe Fügemittel zur Fixierung des absorbierenden Materialabschnitts auf der Oberseite des Basisabdeckmaterials eingesetzt werden (in der Figur nicht dargestellt).

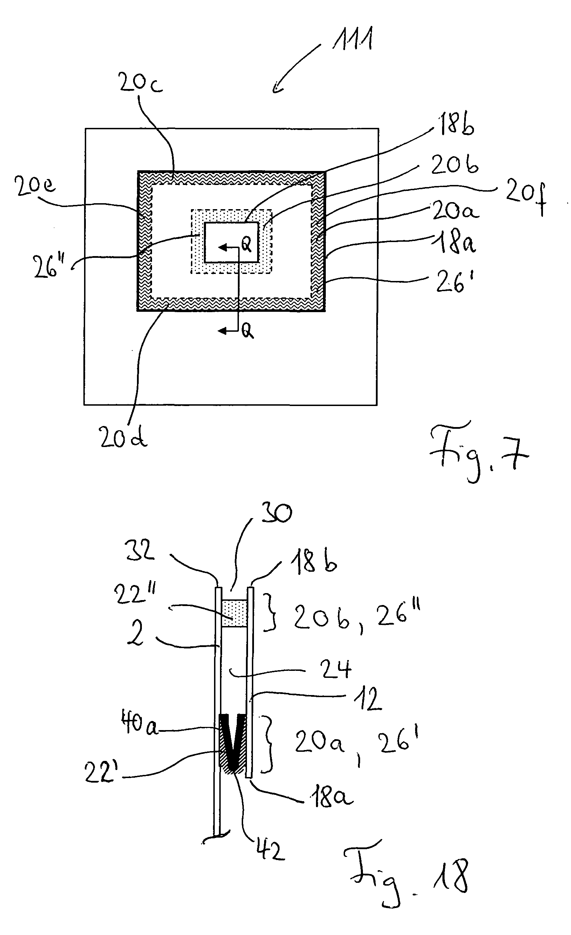

Figur 7 zeigt schematisch eine Draufsicht auf eine OP-Abdeckung 111 mit einer Ausnehmung 30, mit den Merkmalen wie voranstehend unter Figur 4 beschrieben.
Die äußeren Randbereiche 20a, welche an sich durch die Randbereiche 20c, 20d, 20e, 20f gebildet werden und die inneren Randbereiche 20b, können durch den Einsatz von unterschiedlichen Fügemitteln Flüssigkeitsbarrieren 26', 26" bilden. Insbesondere kann die Ausgestaltung derart sein, dass die äußeren Randbereiche 20a im Vergleich zu den inneren Randbereichen 20b unterschiedliche Flüssigkeitsbarrieren aufweisen. Insbesondere vorzugsweise kann die erste Flüssigkeitsbarriere 26' eine im Vergleich zu einer weiteren Flüssigkeitsbarriere 26" stärkere Barriereeigenschaften aufweisen.
Die OP-Abdeckung 111 in der Seitenansicht entlang der Linie Q --- Q ist in der Figur 18 schematisch dargestellt.

Die Figuren 8 -18 zeigen jeweils schematisch die Ausgestaltung des Fügemittels 22' in einer bevorzugten Ausführungsform.

Figur 8 zeigt eine erste bevorzugte Ausführungsform des Fügemittels 22'.
Das Fügemittel 22' ist planar zwischen der Oberseite 6 des Basisabdeckmaterials 2 und der Innenseite 14 des absorbierenden Materialabschnitts 12 angeordnet. Das Fügemittel 22' ist dabei mit seiner in Richtung Randkante 18 des absorbierenden Materialabschnitts 12 weisenden Kante 21 im Wesentlichen bündig zur Randkante 18 des absorbierenden Materialabschnitts angeordnet. Ebenso erstreckt sich das Fügemittel 22' ausgehend von der Randkante 18 über den angrenzenden Randbereich 20. Das Fügemittel 22' ist dabei in dem Randbereich 20 unlösbar fixiert. Durch diese unlösbare Fixierung des absorbierenden Materialabschnitts 12 wird in diesem Randbereich 20 eine Flüssigkeitsbarriere 26' durch das Fügemittel 22' gebildet. Zwischen dem absorbierenden Materialabschnitt 12 und dem Basisabdeckmaterial 2 ist eine Tasche 24 gebildet. Der Randbereich 20 weist dabei eine Breite von 1,5 - 6 cm auf.

Wie in Figur 8 dargestellt, kann das Fügemittel 22' dieser Art eine polymere Schicht 36 umfassen, an deren Oberseiten beidseitig eine adhäsive Beschichtung 38 angebracht wird. Als ein derartiges Fügemittel kann ein beidseitiges Klebebandes eingesetzt werden, dessen beiden klebenden Oberseiten die unlösbare Verbindung zwischen dem Basisabdeckmaterial 2 und dem Materialabschnitt 12 bilden und damit insgesamt die Flüssigkeitsbarriere 26' bereitstellen.

Das Fügemittel in dieser bevorzugten Ausführungsform in der planaren Anordnung kann auch in Form eines Hotmeltstreifens ausgebildet sein (nicht in der Figur dargestellt).

Figur 9 zeigt eine zweite bevorzugte Ausführungsform des Fügemittels 22'.
Analog zur Anordnung des Fügemittels 22' nach Figur 8, ist das Fügemittel 22' planar zwischen der Oberseite 6 des Basisabdeckmaterials 2 und der Innenseite 14 des absorbierenden Materialabschnitts 12 angeordnet. Durch diese unlösbare Fixierung des absorbierenden Materialabschnitts 12 wird in diesem Randbereich 20 eine Flüssigkeitsbarriere 26' durch das Fügemittel 22' gebildet.
Vorteilhaft an dieser Ausführungsform erweist sich, dass die Flüssigkeitsbarriere 26' sich über die Breite des direkt an die Oberseite 6 des Basisabdeckmaterials 2 fixierten Randbereiches 20 in Richtung Tasche 24 hinaus erstreckt.
Dafür weist das Fügemittel 22' an den beiden Oberseiten der polymeren Schicht 36 eine flächenhaft unterschiedliche adhäsive Beschichtung 38 auf. Bei einem Fügemittel dieser Art kann ein doppelseitiges Klebeband eingesetzt werden, bei welchem eine Oberseite vollflächig mit einer adhäsiven Beschichtung versehen ist und die gegenüberliegende Oberseite nur bereichsweise eine adhäsive Beschichtung aufweist. Vorzugsweise ist höchstens die Hälfte, weiter vorzugsweise ist höchstens nur ein Viertel der Breite des Klebebands adhäsiv ausgestaltet. Der entsprechend andere Teil ist dabei nicht klebend ausgestaltet, was dadurch erreicht werden kann, dass keine adhäsive Beschichtung aufgebracht ist oder dass auch eine sich über die gesamte Breite des Bandes erstreckende adhäsive Beschichtung partiell nachträglich inaktiviert wird, so beispielsweise durch eine weitere Lage abgedeckt ist.

Figur 10 zeigt eine dritte bevorzugte Ausführungsform des Fügemittels 22'.
Das Fügemittel 22' ist zwischen der Oberseite 6 des Basisabdeckmaterials 2 und der Innenseite 14 des absorbierenden Materialabschnitts 12 und diese direkt verbindend angeordnet und erstreckt sich weiter über die Randkante 18 des absorbierenden Materialabschnittes 12 hinaus und ist dann zurückgefaltet auf der Oberseite 16 des Materialabschnittes 12 fixiert. Zwischen dem Basisabdeckmaterial 2 und dem absorbierenden Materialabschnitt 12 ist eine Tasche 24 ausgebildet.
Das Fügemittel 22' weist an den beiden Oberseiten der polymeren Schicht 36 eine flächenhaft unterschiedliche adhäsive Beschichtung 38 auf.
Bei einem Fügemittel dieser Art kann ein doppelseitiges Klebeband eingesetzt werden, bei welchem eine Oberseite vollflächig mit einer adhäsiven Beschichtung versehen ist und die gegenüberliegende Oberseite nur bereichsweise eine adhäsive Beschichtung aufweist. Vorzugsweise ist höchstens die Hälfte, weiter vorzugsweise ist höchstens nur ein Viertel der der Breite des Klebebandes adhesiv ausgestaltet. Der entsprechend andere Teil ist dabei nicht klebend ausgestaltet, was dadurch erreicht werden kann, dass keine adhesive Beschichtung aufgebracht ist oder dass auch eine sich über die gesamte Breite des Bandes erstreckende adhäsive Beschichtung partiell nachträglich inaktiviert wird, so beispielsweise durch eine weitere Lage abgedeckt ist.

Figur 11 zeigt eine vierte bevorzugte Ausführungsform des Fügemittels 22'.
Das Fügemittel 22' überfängt den Randbereich auf der Außenseite 16 des absorbierenden Materialabschnitts 12 und erstreckt sich über die Randkante 18 des absorbierenden Materialabschnitts 12 hinaus und ist dann diese Randkante18 übergreifend zurückgefaltet in Richtung Tasche 24 auf der Oberseite 6 des Basisabdeckmaterials fixiert.
Bei einem Fügemittel dieser Art kann ein doppelseitiges und nassstabiles Klebeband mit einer polymeren Schicht 36 eingesetzt werden und zwar dergestalt, dass in Längsrichtung des Klebebands gesehen, die Bereiche mit einer adhäsiven Beschichtung 38 und die nichtadhäsiven Bereiche auf den Oberseiten zueinander versetzt angeordnet sind.

Durch die Anordnung der Fügemittel, so wie in Figuren 10 und 11 beschrieben, wird der absorbierende Materialabschnitt zusätzlich an dieser Randkante von außen durch die mittels des Fügemittels geschaffene Flüssigkeitsbarriere abgedichtet.

Figuren 12 und 13 zeigen eine fünfte bevorzugte Ausführungsform des Fügemittels 22'. Das Fügemittel 22' ist rinnenförmig konfiguriert. Die beiden Schenkel 40a, 40b der Rinne sind ausgehend von der distal angeordneten Faltkante 42 jeweils auf der Oberseite 6 des Basisabdeckmaterials 2 und auf der Innenseite 14 des absorbierenden Materialabschnitts 12 fixiert, so dass sich die Rinne in Richtung auf die Tasche 24 öffnet.

Bei einem Fügemittel dieser Art kann ein einseitiges und nassstabiles Klebeband eingesetzt werden. Die adhäsive Beschichtung 38 der Oberseite der polymeren Lage 36 kann dabei vorteilhafterweise im Bereich der vorgesehenen Faltkante 42 unterbrochen sein, im Sinne dass in diesem Bereich kein adhäsiver Beschichtungsauftrag vorliegt bzw. auch dass die an sich vollflächige adhäsive Beschichtung in diesem Bereich inaktiviert ist, bspw. durch eine weitere Lage abgedeckt ist.

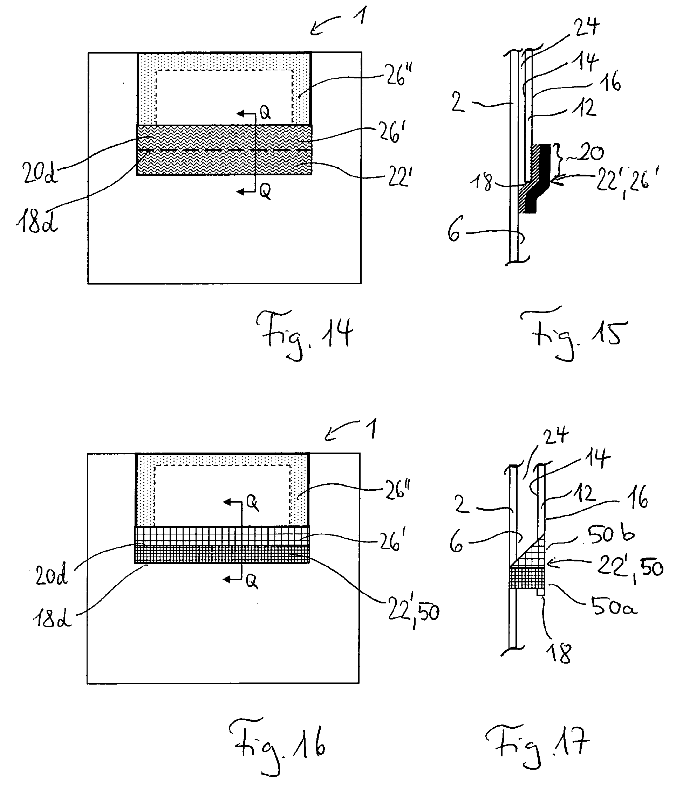

Figuren 14 und 15 zeigen eine sechste bevorzugte Ausführungsform des Fügemittels 22'.

Die Figur 14 zeigt schematisch eine Draufsicht auf eine, wie in Figur 1 beschriebene OP-Abdeckung 1 mit der bevorzugten Ausführung, dass zumindest der an die untere Randkante 18d angrenzende untere Randbereich 20d des absorbierenden Materialabschnitts 12 eine Flüssigkeitsbarriere 26' aufweist, welche durch das in Figur 15 näher dargestellte Fügungsmittel 22' (Seitenansicht entlang der Linie Q ---Q) bereitgestellt wird.
Die übrigen Randbereiche weisen eine weitere Flüssigkeitsbarriere 26" auf.

Das Fügemittel 22' in einer sechsten bevorzugten Ausführungsform überfängt sowohl einen Randbereich 20 auf der Außenseite 16 des absorbierenden Materialabschnitts 12 als auch einen daran unmittelbar angrenzenden Teil der Oberseite 6 des Basisabdeckmaterials 2.

Bei einem Fügemittel dieser Art kann ein einseitiges und nassstabiles Klebeband eingesetzt werden.

Figuren 16 und 17 zeigen eine siebte bevorzugte Ausführungsform des Fügemittels 22'. Die Figur 16 zeigt schematisch eine Draufsicht auf eine, wie in Figur 1 beschriebene OP-Abdeckung 1 mit der bevorzugten Ausführung, dass zumindest der an die untere Randkante 18d angrenzende untere Randbereich 20d des absorbierenden Materialabschnitts 12 eine Flüssigkeitsbarriere 26' aufweist, welche durch das in Figur 17 näher dargestellte Fügungsmittel 22' (Seitenansicht entlang der Linie Q ---Q) bereitgestellt wird.
Die übrigen Randbereiche weisen eine weitere Flüssigkeitsbarriere 26" auf.

Das Fügemittel 22' ist in Form von Schweißstellen ausgebildet.
Die Schweißstellen können dabei Ultraschallschweißstellen, thermisch Schweißstellen (thermobonding) oder Kalanderschweißstellen sein.
Die Schweißstellen sind dabei in Form eines Schweißmusters 50 angeordnet.
Das Schweißmuster kann sich dabei aus der Anordnung der Schweißstellen und deren Abstand zueinander, also der Schweißstellendichte ergeben. Ebenso kann das Schweißmuster 50 aus unterschiedlicher Tiefe der Schweißstellen resultieren.
Das Fügemittel 22' in dieser siebten bevorzugten Ausführungsform erstreckt sich zumindest über den Randbereich 20d. Das Fügemittel 22' kann sich auch noch über die Randkante 18d und den daran angrenzenden Teil des Basisabdeckmaterials 2 erstrecken (nicht in der Figur dargestellt).
Das Schweißmuster 50 ist derart gestaltet, dass es ausgehend von einer Randkante 18 in Richtung Tasche 24 ein abnehmendes Profil hinsichtlich Bindestellentiefe und/oder Bindestellendichte und/oder Bindestellenstärke aufweist. Ein erster Teilbereich des Randbereichs, welcher proximal zur Randkante 18 angeordnet ist, weist dabei ein erstes Schweißmuster 50a auf. Der weitere, in Richtung Tasche 24 weisende Teilbereich weist dabei ein zweites Schweißmuster 50b auf. Das erste Schweißmuster 50a weist dabei Schweißstellen auf, welche eine unlösbare Fixierung zwischen dem absorbierenden Materialabschnitt 12 ausgehend von seiner Oberseite 16 und dem Basisabdeckmaterial 2 bilden. Das zweite Schweißmuster 50b ist dabei derart, dass lediglich der absorbierende Materialabschnitt, insbesondere nur die Oberseite 16 des absorbierenden Materialabschnitts Schweißstellen aufweisen.

Figur 18 zeigt schematisch die Seitenansicht, entlang der Linie Q --- Q entnommen aus Figur 7, und zwar die an einem äußeren Randbereich 20a und an einem inneren Randbereich 20b eingebrachten Fügemittel 22', 22" in unterschiedlichen Ausführungsformen. Die inneren Randkanten 18b des absorbierenden Materialabschnitts 1 sind kantenbündig um die Ausnehmung 30 mit deren Kanten 32 angeordnet.
Am äußeren Randbereich 20a ist ein Fügemittel 22' in einer rinnenförmigen Konfiguration, so wie zu Figur 12 beschrieben, eingebracht. Am inneren Randbereich 20b ist ein Fügemittel 22" in einer planaren Konfiguration, so wie zu Figur 8 beschrieben eingebracht. Durch jedes Fügemittel 22', 22" ist der absorbierende Materialabschnitt 12 in den jeweiligen Randbereichen 20a, 20b unlösbar auf der Oberseite 6 des Basisabdeckmaterials 2 unter Ausbildung der Tasche 24 fixiert. Die durch diese Fügemittel 22', 22" gebildete Flüssigkeitsbarrieren 26', 26" unterscheiden sich.
Die am äußeren Randbereich 20a durch das rinnenförmige Fügemittel 22' bereitgestellte erste Flüssigkeitsbarriere 26' stellt eine stärkere Flüssigkeitsbarriere dar als die am inneren Randbereich 20b durch das planare Fügemittel 22" bereitgestellte weitere Flüssigkeitsbarriere 26".

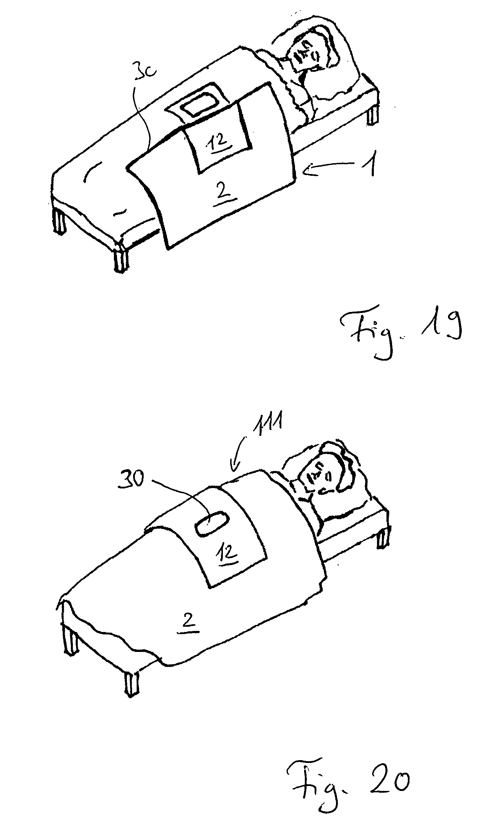

Figuren 19 und 20 zeigen schematisch erfindungsgemäße OP-Abdeckungen in ihrer im Gebrauch möglichen Anordnung.
Figur 19 zeigt eine OP-Abdeckung in der Ausführung nach Figur 1. Der absorbierende Materialabschnitt 12 ist dabei im Wesentlichen bündig am oberen Ende 3c der OP-Abdeckung angeordnet.
Figur 19 zeigt eine OP-Abdeckung in der Ausführung nach Figur 3. Der absorbierende Materialabschnitt 12 ist dabei im Wesentlichen bündig an den Kanten der Ausnehmung 30 der OP-Abdeckung 111 angeordnet.

Vorteilhafte Ausführungen an erfindungsgemäßen OP-Abdeckungen :
Die in Figur 5 dargestellte OP-Abdeckung 1 weist erkennbar ein flüssigkeitsundurchlässiges Basisabdeckmaterial 2 mit einer im Gebrauch zum Patienten gerichteten Unterseite 4 und mit einer Oberseite 6 auf, wobei diese Oberseite 6 ihrerseits eine absorbierende Schicht hat.
Das Basisabdeckmaterial besteht aus einem Vlies-Folien-Verbund, wobei die zur Oberseite 6 gerichtete Schicht des Vlieses aus einem hydrophilisiertem Spunbond-Vlies, insbesondere aus einem hydrophilisiertem PP-Spunbond-Vlies besteht. Das Flächengewicht der Spunbondvlies-Lage beträgt 25 - 35 g/m². Die Absorptionskapazität AK dieses Spunbond-Vlieses beträgt nach der oben beschriebenen Methode 120 - 180 g/m².
Die Oberseite 6 weist einen ersten absorbierenden Bereich 8 und einen zweiten absorbierenden Bereich 10 auf. Dieser zweite absorbierende Bereich 10 ist seinerseits durch einen rechteckförmigen Materialabschnitt 12 mit einer Innenseite und einer Außenseite, welcher Flüssigkeiten absorbierend und flüssigkeitsdurchlässig ist, überfangen. Dieser absorbierende Materialabschnitt12 ist aus einem hydrophilisiertem SMS-Vlies, insbesondere aus einem hydrophilisiertem PP-SMS-Vlies mit einem Flächengewicht von 50 - 60 g/m² gebildet. Die Absorptionskapazität dieses SMS-Vlieses beträgt nach der oben beschriebenen Methode 220 - 330 g/m².
Der absorbierende Materialabschnitt 12 mit Randkanten 18 und an die Randkanten angrenzende Randbereichen 20 ist in seinen Randbereichen vollumfänglich auf der Oberseite 6 des Basisabdeckmaterials unlösbar fixiert. Außerhalb dieser Randbereiche 20 ist der absorbierende Materialabschnitt 12 im Wesentlichen nicht mit dem Basisabdeckmaterial verbunden. Somit ist zwischen dem absorbierenden Materialabschnitt 12 und dem flüssigkeitsundurchlässigem Basisabdeckmaterial 2 eine Tasche ausgebildet. Die OP-Abdeckung erstreckt sich in Längsrichtung LR zwischen einem oberen Ende 3c und einem unteren Ende 3d, wobei der absorbierende Materialabschnitt näher am oberen Ende 3c der OP-Abdeckung als an dem unteren Ende 3d der OP-Abdeckung angeordnet.
In dieser Anordnung weist auch der Materialabschnitt 12 entsprechend eine obere Randkante 18c, eine untere Randkante18d und davon anschließende seitliche Randkanten 18e, 18f und entsprechende an diese Randkanten 18c, 18d, 18e, 18f angrenzende Randbereiche 20c, 20d, 20e, 20f auf.
Der Materialabschnitt ist mit seiner oberen Randkante 18c im Wesentlichen bündig zum oberen Ende 3c der OP-Abdeckung angeordnet.
Die Randbereiche 20c, 20d, 20e, 20f weisen zumindest abschnittsweise eine Flüssigkeitsbarriere 26', 26" auf, wobei die Flüssigkeitsbarriere 26', 26" durch ein Fügemittel gebildet ist, welches den Materialabschnitt unlösbar auf der Oberseite des Basisabdeckmaterials fixiert.

Es werden unterschiedliche Fügemittel 22', 22" für den unteren Randbereich 20d und für die weiteren Randbereiche 20c, 20e, 20f eingesetzt, so dass der untere Randbereich eine erste Flüssigkeitsbarriere 26' und die weiteren Randbereiche eine weitere Flüssigkeitsbarriere 26" aufweisen.

Die Fügelmittel 22', 22" werden vorzugsweise in folgenden Kombinationen eingesetzt:
In einer ersten bevorzugten Variante der OP-Abdeckung ist das Fügemittel 22' im unteren Randbereich 20d rinnenförmig konfiguriert, entsprechend der fünften Ausführungsform des Fügemittels, so wie zu Figuren 12 und 13 beschrieben ist.
Als rinnenförmiges Fügemittel wird hierbei ein einseitiges und nässestabiles Klebeband (wie beispielsweise MED 248 vertrieben durch die Fa. Köster, Altendorf, Deutschland) eingesetzt.
Das einseitige Klebeband ist dazu in seiner Längsrichtung unter Ausbildung einer Faltkante 42 und zwei Schenkeln 40a, 40b gefaltet. Die Faltkante 42 kann dabei mittig gelegen sein. Die Faltkante kann in Bezug auf die Breite des Klebebandes auch exzentrisch gelegen sein. Das Klebeband wird derart angebracht, so dass die Faltkante 42 im Wesentlich bündig mit der unteren Randkante 18d des absorbierenden Materialabschnitts 12 abschließt und die beiden Schenkel 40a, 40b mit ihrer jeweiligen adhäsiven Oberseite auf der Innenseite 14 des absorbierenden Materialabschnitts und auf der Oberseite 6 des Basisabdeckmaterials fixiert werden. Die Rinne öffnet sich in Richtung auf die Tasche 24. Damit wird im unteren Randbereich 20d eine erste Flüssigkeitsbarriere 26' gebildet.

In einer zweiten bevorzugten Variante der OP-Abdeckung ist das Fügemittel 22' im unteren Randbereich 20d entsprechend der vierten bevorzugten Ausführungsform des Fügemittels , so wie zu Figur 11 beschrieben, gestaltet und fixiert.

In einer dritten bevorzugten Variante der OP-Abdeckung ist das Fügemittel 22' im unteren Randbereich 20d entsprechend der sechsten bevorzugten Ausführungsform des Fügemittels, so wie zu Figur 15 beschrieben, gestaltet und fixiert.
Als Fügemittel, das sowohl die Außenseite des absorbierenden Materialabschnittes als auch noch an den Randbereich daran unmittelbar angrenzenden Teil der Oberseite 6 des Basisabdeckmaterials 2 überfängt, kann ein einseitig und nassstabiles Klebeband mit seiner adhäsiven Oberseite zur Außenseite 16 und Oberseite 6 gerichtet, eingesetzt werden.

In einer vierten bevorzugten Variante der OP-Abdeckung ist das Fügemittel 22' im unteren Randbereich 20d entsprechend der siebten bevorzugten Ausführungsform des Fügemittels, so wie zu Figuren 16 und 17 beschrieben, gestaltet und fixiert.

In einer fünften bevorzugten Variante der OP-Abdeckung ist das Fügemittel 22' im unteren Randbereich 20d entsprechend der vorangehend näher erläuterten zweiten Ausführungsform des Fügemittels, so wie zu Figur 9 beschrieben, gestaltet und fixiert.

In einer sechsten bevorzugten Variante der OP-Abdeckung ist das Fügemittel 22' im unteren Randbereich 20d entsprechend der vorangehend näher erläuterten dritten Ausführungsform des Fügemittels, so wie zu Figur 10 beschrieben, gestaltet und fixiert.

Für alle genannten Varianten der OP-Abdeckung werden in übrigen Randbereichen 20c, 20e, 20f vorzugsweise Fügemittel 22', 22" eingesetzt, welche planar zwischen der Oberseite des Basisabdeckmaterials 2 und der Innenseite des absorbierenden Materialabschnitts, entsprechend der ersten bevorzugten Ausführungsform nach Figur 8 oder auch vorzugsweise entsprechend der zweiten bevorzugten Ausführungsform nach Figur 9, angeordnet sind.
Diese Randbereiche 20c, 20e, 20f werden mittels eines Hotmeltklebers fixiert. Diese Randbereiche 20c, 20e, 20f können aber auch mittels eines doppelseitigen nässestabilen Klebebands fixiert werden.

Die in Figur 6 dargestellte OP-Abdeckung 1 unterscheidet sich von der in Figur 5 dargestellten und voranstehend beschriebenen OP-Abdeckung und ihren Varianten dahingehend, dass die in dem unteren Randbereich 20d eingesetzten Fügemittel 22' auch noch in den an den unteren Randbereich 20d jeweils seitlich daran anschließenden Randbereiche 20e, 20f zur unlösbaren Fixierung des absorbierenden Materialabschnittes an die Oberseite des Basisabdeckmaterials eingesetzt werden.
Entsprechend wird nur der obere Randbereich 20c vorzugsweise mit einem planar zwischen der Oberseite 6 des Basisabdeckmaterials 2 und der Innenseite 14 des absorbierenden Materialabschnitts, entsprechend der ersten Ausführungsform nach Figur 8, also in Form eines Hotmeltklebers oder auch eines zweiseitigen Klebebandes unter Bildung der weiteren Flüssigkeitsbarriere 26" fixiert.
Als Hotmeltkleber kann beispielsweise Nolax M11.123, vertrieben durch die Fa. Collano, Sempach, Schweiz eingesetzt werden.

Ebenso ist auch denkbar, dass alle Randbereiche 20c, 20d, 20e, 20f eine gleiche Flüssigkeitsbarriere 26' aufweisen. Diese Flüssigkeitsbarriere 26' wird durch ein Fügemittel 22' gebildet ist, welches den Materialabschnitt 12 entlang aller Randbereiche 20c, 20d, 20e, 20f unlösbar auf der Oberseite des Basisabdeckmaterials fixiert.
Hierfür sind alle beschriebenen Ausführungsformen des Fügemittels denkbar.

Auch wenn nicht näher dargestellt, so können auch OP-Abdeckungen 11, 111 mit einer Ausnehmung 30 (wie schematisch in den Figuren 3 und 4 dargestellt) alle Merkmale der zuvor beschriebenen OP-Abdeckung in Bezug auf die Materialeigenschaften/Zusammensetzung des Basisabdeckmaterials und des absorbierenden Materialabschnitts aufweisen.

Die an ihren jeweiligen äußeren und inneren Randbereichen 20a, 20b eingesetzten Fügemittel 22', 22" unterscheiden sich, so dass entsprechend im äußeren und im inneren Randbereich zumindest abschnittsweise eine erste 26' und eine weitere Flüssigkeitsbarriere 26" gebildet wird.
Die äußeren und inneren Randbereiche ihrerseits können wieder in den oberen, unteren und in den an den unteren Randbereich seitlich anschließenden Randbereiche mit unterschiedlichen Fügemittel, unter Ausbildung weiterer Flüssigkeitsbarrieren, fixiert sein.

## Patentansprüche

1. Wegwerfbare OP-Abdeckung (1, 11, 111) umfassend ein flüssigkeitsundurchlässiges Basisabdeckmaterial (2) mit einer in Gebrauch zum Patienten gerichteten Unterseite (4) und einer Oberseite (6), wobei die Oberseite eine absorbierende Schicht aufweist, und wobei sich die OP-Abdeckung in einer Längsrichtung LR zwischen einem oberen Ende (3c) und einem unteren Ende (3d) erstreckt, und wobei die Oberseite (6) einen ersten absorbierenden Bereich (8) und mindestens einen zweiten absorbierenden Bereich (10) aufweist, und wobei der zweite absorbierende Bereich (10) von einem Materialabschnitt (12) mit Randkanten (18a, 18b, 18c, 18d, 18e, 18f) und an die Randkanten (18a, 18b, 18d, 18e, 18f) angrenzenden Randbereichen (20a, 20b, 20c, 20d, 20e, 20 f) überfangen ist, welcher Flüssigkeiten absorbierend und flüssigkeitsdurchlässig ist und eine Innenseite (14), eine Außenseite (16) aufweist, wobei diejenige verbleibende Fläche der Oberseite (6) des Basisabdeckmaterials (2), die nicht durch den absorbierenden Materialabschnitt (12) abgedeckt ist, den ersten absorbierenden Bereich (8) bildet, wobei der absorbierende Materialabschnitt (12) in den Randbereichen (20a, 20b, 20c, 20d, 20e, 20f) vollumfänglich auf der Oberseite (6) des Basisabdeckmaterials (2) unlösbar fixiert ist und der absorbierende Materialabschnitt (12) außerhalb der Randbereiche (20a, 20b, 20c, 20d, 20e, 20f) im Wesentlichen nicht mit dem Basisabdeckmaterial (2) verbunden ist, derart, dass zwischen dem absorbierenden Materialabschnitt (12) und dem flüssigkeitsundurchlässigen Basisabdeckmaterial (2) eine Tasche (24) gebildet ist, und wobei mindestens einer der Randbereiche (20a, 20b, 20c, 20d, 20e, 20 f) des absorbierenden Materialabschnitts (12) zumindest abschnittsweise eine Flüssigkeitsbarriere (26', 26") aufweist, wobei die Flüssigkeitsbarriere durch ein Fügemittel (22', 22") gebildet ist, welches den absorbierenden Materialabschnitt (12) unlösbar auf der Oberseite (6) des Basisabdeckmaterials (2) fixiert, **dadurch gekennzeichnet, dass** das Basisabdeckmaterial (2) aus einem ein- oder mehrlagigen Vliesstoff oder einem Vlies-Folien-Verbund gebildet ist und dass der absorbierende Materialabschnitt (12) durch einen ein- oder mehrlagigen Vliesstoff gebildet ist, wobei der zweite absorbierende Bereich durch die flächenhafte Erstreckung des absorbierenden Materialabschnittes und durch die vom absorbierenden Materialabschnitt überfangene flächenhafte Erstreckung des darunter liegenden Basisabdeckmaterials gebildet ist.

2. Wegwerfbare OP-Abdeckung nach Anspruch 1, **dadurch gekennzeichnet, dass** die OP-Abdeckung innerhalb des zweiten absorbierenden Bereiches (10) zumindest eine Ausnehmung (30) aufweist.

3. Wegwerfbare OP-Abdeckung nach Anspruch 2, **dadurch gekennzeichnet, dass** der zweite absorbierende Bereich (10) die Ausnehmung (30) unmittelbar umgibt und an diese angrenzt und der absorbierende Materialabschnitt (12) den Kanten (32) der Ausnehmung (30) zugeordnete innere Randkanten (18b) und innere Randbereiche (20b) sowie äußere Randkanten (18a) und äußere Randbereiche (20a) aufweist, wobei insbesondere die inneren Randkanten (18b) des absorbierenden Materialabschnitts (12) im Wesentlichen bündig zu den Kanten (32) der Ausnehmung (30) angeordnet sind.

4. Wegwerfbare OP-Abdeckung nach einem der vorstehenden Ansprüche, **dadurch gekennzeichnet, dass** ein unterer Randbereich (20d) des Materialabschnitts (12) eine Flüssigkeitsbarriere (26', 26") aufweist, wobei insbesondere die an einen unteren Randbereich (20d) des Materialabschnitts (12) sich jeweils seitlich daran anschließenden Randbereiche (20e, 20f) des Materialabschnitts (12) eine Flüssigkeitsbarriere (26', 26") aufweisen.

5. Wegwerfbare OP-Abdeckung nach einem der vorstehenden Ansprüche, **dadurch gekennzeichnet, dass** nur die äußeren Randbereiche (20a) des Materialabschnitts (12) eine Flüssigkeitsbarriere (26', 26") aufweisen.

6. Wegwerfbare OP-Abdeckung nach einem der vorstehenden Ansprüche, **dadurch gekennzeichnet, dass** unterschiedliche Fügemittel (22', 22") eingesetzt werden, derart dass eine erste und zumindest eine weitere Flüssigkeitsbarriere (26', 26") gebildet wird.

7. Wegwerfbare OP-Abdeckung nach einem der vorstehenden Ansprüche, **dadurch gekennzeichnet, dass** die Fügemittel (22', 22") entnommen sind aus der Gruppe Hotmeltkleber, ein- oder mehrseitiges Klebeband, Schweißstellen, insbesondere Ultraschallschweißstellen, thermische Schweißstellen, Kalanderschweißstellen.

8. Wegwerfbare OP-Abdeckung nach einem der vorstehenden Ansprüche, **dadurch gekennzeichnet, dass** das Fügemittel (22', 22") planar zwischen der Oberseite (6) der Basisabdeckmaterials (2) und der Innenseite (14) des absorbierenden Materialabschnitts (12) angeordnet ist.

9. Wegwerfbare OP-Abdeckung nach einem der vorstehenden Ansprüche, **dadurch gekennzeichnet, dass** das Fügemittel (22', 22") zwischen der Oberseite (6) des Basisabdeckmaterials (2) und der Innenseite (14) des absorbierenden Materialabschnitts (12) und diese direkt verbindend angeordnet ist und sich über die Randkante (18a, 18b, 18c, 18d, 18e, 18f) des absorbierenden Materialabschnittes (12) hinaus erstreckend und dann zurückgefaltet auf dessen Oberseite (16) fixiert ist.

10. Wegwerfbare OP-Abdeckung nach einem der vorstehenden Ansprüche, **dadurch gekennzeichnet, dass** das Fügemittel (22', 22") an der Oberseite (6) des Basisabdeckmaterials (2) angeordnet ist und sich über die Randkante (18a, 18b, 18c, 18d, 18e, 18f) des absorbierenden Materialabschnittes (12) hinaus erstreckend und dann zurückgefaltet auf dessen Oberseite (16) fixiert ist.

11. Wegwerfbare OP-Abdeckung nach einem der vorstehenden Ansprüche, **dadurch gekennzeichnet, dass** das Fügemittel (22', 22") rinnenförmig konfiguriert ist und die beiden Schenkel (40a, 40b) der Rinne ausgehend von der distal angeordneten Faltkante (42) jeweils auf der Oberseite (6) des Basisabdeckmaterials (2) und auf der Innenseite (14) des absorbierenden Materialabschnitts (12) fixiert sind, derart, dass sich die Rinne in Richtung auf die Tasche (24) öffnet.

12. Wegwerfbare OP-Abdeckung nach einem der vorstehenden Ansprüche, **dadurch gekennzeichnet, dass** das Fügemittel (22', 22") sowohl einen Randbereich (20a, 20b, 20c, 20d, 20e, 20f) auf der Außenseite (16) des absorbierenden Materialabschnitts (12) als auch einen daran unmittelbar angrenzenden Teil der Oberseite (6) des Basisabdeckmaterials (2) überfängt.

13. Wegwerfbare OP-Abdeckung nach Anspruch 7, **dadurch gekennzeichnet, dass** die Schweißstellen in Form eines Schweißmuster angeordnet sind, wobei das Schweißmuster ausgehend von einer Randkante (18a, 18b, 18c, 18d, 18e, 18f) und den angrenzenden Randbereich (20a, 20b, 20c, 20d, 20e, 20f) überfangend in Richtung Tasche (24) ein abnehmendes Profil hinsichtlich Bindestellentiefe und/oder Bindestellendichte und/oder Bindestellenstärke aufweist.

14. Wegwerfbare OP-Abdeckung nach einem der vorstehenden Ansprüche, **dadurch gekennzeichnet, dass** die Flüssigkeitsbarriere (26', 26") sich über die Breite des fixierten Randbereiches (20a, 20b, 20c, 20d, 20e, 20f) in Richtung Tasche hinaus erstreckt.

15. Wegwerfbare OP-Abdeckung nach einem der vorstehenden Ansprüche, **dadurch gekennzeichnet, dass** die fixierten Randbereiche (20a, 20b, 20c, 20d, 20e, 20f) eine Fläche von 2 - 45% , insbesondere von 2 - 35 %, weiter insbesondere von 2- 30%, bezogen auf die Gesamtfläche des absorbierenden Materialabschnitts (12) einnehmen.

16. Wegwerfbare OP-Abdeckung nach einem der vorstehenden Ansprüche, **dadurch gekennzeichnet, dass** der absorbierende Materialabschnitt (12) eine größere Absorptionskapazität AK aufweist als das den ersten absorbierenden Bereich (8) bildende Material, insbesondere dass der absorbierende Materialabschnitt (12) eine um mindestens 25% größere, insbesondere eine um mindestens 50% größere, insbesondere eine um mindestens 100 % größere, insbesondere eine um mindestens 150% größere, weiter insbesondere eine um mindestens 200% größere, weiter insbesondere eine um mindestens 250% weiter insbesondere eine um mindestens 300 % größere Absorptionskapazität AK aufweist als das den ersten Bereich bildende Material.

17. Wegwerfbare OP-Abdeckung nach einem der vorstehenden Ansprüche, **dadurch gekennzeichnet, dass** der absorbierende Materialabschnitt eine relative Absorptionskapazität AK von mindestens 150 g/m², insbesondere mindestens 200 g/m², insbesondere mindestens 250 g/m², insbesondere mindestens 300 g/m², insbesondere mindestens 350 g/m² aufweist, mit der Absorptionskapazität AK gemessen nach der beschriebenen Methode.

18. Wegwerfbare OP-Abdeckung nach einem der vorstehenden Ansprüche, **dadurch gekennzeichnet, dass** das den ersten Bereich bildende Material eine Absorptionskapazität AK von 50 - 250 g/m², insbesondere 80 - 220 g/m², weiter insbesondere 100 - 200 g/m² aufweist, mit der Absorptionskapazität AK gemessen nach der beschriebenen Methode.

19. Wegwerfbare OP-Abdeckung nach einem der vorstehenden Ansprüche, **dadurch gekennzeichnet, dass** der zweite absorbierende Bereich (10) eine flächenhafte Erstreckung von 2 - 65 %, vorzugsweise 2 - 55 %, weiter vorzugsweise 2 - 45 % bezogen auf die Gesamtfläche des Basisabdeckmaterials aufweist.

20. Wegwerfbare OP-Abdeckung nach einem der vorstehenden Ansprüche, **dadurch gekennzeichnet, dass** der absorbierende Materialabschnitt (12) aus einem hydrophilisiertem Vliesstoff mit ein oder mehreren Lagen aus Spunbond und/oder Meltblown besteht.

21. Wegwerfbare OP-Abdeckung nach einem der vorstehenden Ansprüche, **dadurch gekennzeichnet, dass** der absorbierende Materialabschnitt (12) ein Flächengewicht von vorzugsweise 25 - 100g/m², insbesondere 35 - 80 g/m², weiter insbesondere 45 - 65 g/m², weiter vorzugsweise von 50 - 60 g/m² aufweist.

22. Wegwerfbare OP-Abdeckung nach einem der vorstehenden Ansprüche, **dadurch gekennzeichnet, dass** das Basisabdeckmaterial (2) aus einem Laminat aus einer oder mehreren Spunbondvlies- und/oder Meltblownvlieslagen oder aus einem zweilagigen Vlies-Folien-Verbund mit einem Vliesstoff aus einen hydrophilisiertem Spunbondvlies mit der Unterseite aus Folienmaterial besteht.

## Claims

1. Disposable surgical drape (1, 11, 111) comprising a liquid-impermeable basic drape material (2) having a lower side (4) which when in use faces the patient, and an upper side (6), wherein the upper side has an absorbent layer, and wherein the surgical drape in a longitudinal direction LR extends between an upper end (3c) and a lower end (3d), and wherein the upper side (6) has a first absorbent region (8) and at least one second absorbent region (10), and wherein the second absorbent region (10) is traversed by a material portion (12) having peripheral edges (18a, 18b, 18c, 18d, 18e, 18f) and by peripheral regions (20a, 20b, 20c, 20d, 20e, 20f) which are contiguous to the peripheral edges (18a, 18b, 18d, 18e, 18f), said material portion (12) being absorbent and liquid-permeable and having an inner side (14), an outer side (16), wherein the remaining area of the upper side (6) of the basic drape material (2) that is not covered by the absorbent material portion (12) forms the first absorbent region (8), wherein the absorbent material portion (12) in the peripheral regions (20a, 20b, 20c, 20d, 20e, 20f) along the entire perimeter is unreleasably fixed to the upper side (6) of the basic drape material (2), and the absorbent material portion (12) outside the peripheral regions (20a, 20b, 20c, 20d, 20e, 20f) is not substantially connected to the basic drape material (2) in such a manner that a pocket (24) is formed between the absorbent material portion (12) and the liquid-impermeable basic drape material (2), and wherein at least one of the peripheral regions (20a, 20b, 20c, 20d, 20e, 20f) of the absorbent material portion (12) at least in portions has a liquid barrier (26', 26''), wherein the liquid barrier is formed by a joining means (22', 22'') which fixes the absorbent material portion (12) unreleasably to the upper side (6) of the basic drape material (2), **characterized in that** the basic drape material (2) is formed from a single or multiple layer non-woven material or from a non-woven/film composite, and **in that** the absorbent material portion (12) is formed by a single or multiple layer non-woven material, wherein the second absorbent region is formed by the planar extent of the absorbent material portion and by that planar extent of the underlying basic drape material that is traversed by the absorbent material portion.

2. Disposable surgical drape according to Claim 1, **characterized in that** the surgical drape has at least one clearance (30) within the second absorbent region (10).

3. Disposable surgical drape according to Claim 2, **characterized in that** the second absorbent region (10) directly surrounds the clearance (30) and is contiguous to the latter, and the absorbent material portion (12) has inner peripheral edges (18b) and inner peripheral regions (20b) as well as outer peripheral edges (18a) and outer peripheral regions (20a), said edges and regions being assigned to the edges (32) of the clearance (30), wherein in particular the inner peripheral edges (18b) of the absorbent material portion (12) are disposed so as to be substantially flush with the edges (32) of the clearance (30).

4. Disposable surgical drape according to one of the preceding claims, **characterized in that** a lower peripheral portion (20d) of the material portion (12) has a liquid barrier (26', 26''), wherein in particular those peripheral regions (20e, 20f) of the material portion (12) that in each case laterally adjoin a lower peripheral region (20d) of the material portion (12) have a liquid barrier (26', 26'').

5. Disposable surgical drape according to one of the preceding claims, **characterized in that** only the outer peripheral regions (20a) of the material portion (12) have a liquid barrier (26', 26'').

6. Disposable surgical drape according to one of the preceding claims, **characterized in that** various joining means (22', 22'') are employed in such a manner that a first and at least one further liquid barrier (26', 26'') are formed.

7. Disposable surgical drape according to one of the preceding claims, **characterized in that** the joining means (22', 22'') are retrieved from the group including hot-melt adhesives, single-sided or double-sided tapes, welded spots, in particular ultrasonically welded spots, thermally welded spots, calender-welded spots.

8. Disposable surgical drape according to one of the preceding claims, **characterized in that** the joining means (22, 22'') is disposed in a planar manner between the upper side (6) of the basic drape material (2) and the inner side (14) of the absorbent material portion (12).

9. Disposable surgical drape according to one of the preceding claims, **characterized in that** the joining means (22', 22'') is disposed between the upper side (6) of the basic drape material (2) and the inner side (14) of the absorbent material portion (12) so as to directly connect said sides, and extends beyond the peripheral edge (18a, 18b, 18c, 18d, 18e, 18f) of the absorbent material portion (12) and thereafter is fixed by being folded back onto the upper side (16) of said absorbent material portion (12).

10. Disposable surgical drape according to one of the preceding claims, **characterized in that** the joining means (22', 22") is disposed on the upper side (6) of the basic drape material (2), and extends beyond the peripheral edge (18a, 18b, 18c, 18d, 18e, 18f) of the absorbent material portion (12) and thereafter is fixed by being folded back onto the upper side (16) of said absorbent material portion (12).

11. Disposable surgical drape according to one of the preceding claims, **characterized in that** the joining émeans (22', 22") is configured in a channel-shaped manner, and the two legs (40a, 40b) of the channel, proceeding from the distally disposed fold edge (42), are each fixed on the upper side (6) of the basic drape material (2) and on the inner side (14) of the absorbent material portion (12) in such a manner that the channel opens out towards the pocket (24).

12. Disposable surgical drape according to one of the preceding claims, **characterized in that** the joining means (22', 22'') both has a peripheral region (20a, 20b, 20c, 20d, 20e, 20f) on the outer side (16) of the absorbent material portion (12) as well as traverses a part of the upper side (6) of the basic drape material (2) that is directly contiguous to said peripheral region (20a, 20b, 20c, 20d, 20e, 20f).

13. Disposable surgical drape according to Claim 7, **characterized in that** the welded spots are disposed in the form of a welding pattern, wherein the welding pattern, proceeding from a peripheral edge (18a, 18b, 18c, 18d, 18e, 18f) and traversing the contiguous peripheral region (20a, 20b, 20c, 20d, 20e, 20f), towards the pocket (24) in terms of the depth of the joining spot and/or the density of the joining spot and/or the thickness of the joining spot has a decreasing profile.

14. Disposable surgical drape according to one of the preceding claims, **characterized in that** the liquid barrier (26', 26'') extends beyond the width of the fixed peripheral region (20a, 20b, 20c, 20d, 20e, 20f) towards the pocket.

15. Disposable surgical drape according to one of the preceding claims, **characterized in that** the fixed peripheral regions (20a, 20b, 20c, 20d, 20e, 20f) occupy an area of 2 to 45%, particularly of 2 to 35%, more particularly of 2 to 30% of the entire area of the absorbent material portion (12).

16. Disposable surgical drape according to one of the preceding claims, **characterized in that** the absorbent material portion (12) has a larger absorption capacity AK than that material that forms the first absorbent region (8), in particular **in that** the absorbent material portion (12) has an absorption capacity AK that is larger by at least 25%, particularly larger by at least 50%, particularly larger by at by at least 100%, particularly larger by at least 150%, more particularly larger by at least 200%, more particularly larger by at least 250%, more particularly larger by at least 300% than that material that forms the first region.

17. Disposable surgical drape according to one of the preceding claims, **characterized in that** the absorbent material portion has a relative absorption capacity AK of at least 150 g/m², particularly at least 200 g/m², particularly at least 250 g/m², particularly at least 300 g/m², particularly at least 350 g/m², the absorption capacity AK being measured according to the method described.

18. Disposable surgical drape according to one of the preceding claims, **characterized in that** that material that forms the first region has an absorption capacity AK of 50 to 250 g/m², particularly 80 to 220 g/m², more particularly 100 to 200 g/m², the absorption capacity AK being measured according to the method described.

19. Disposable surgical drape according to one of the preceding claims, **characterized in that** the second absorbent region (10) has a planar extent of 2 to 65%, preferably 2 to 55%, more preferably 2 to 45% of the entire area of the basic drape material.

20. Disposable surgical drape according to one of the preceding claims, **characterized in that** the absorbent material portion (12) is composed of a hydrophilicized non-woven material having one or a plurality of layers of spun-bonded and/or melt-blown material.

21. Disposable surgical drape according to one of the preceding claims, **characterized in that** the absorbent material portion (12) has a mass per unit area of preferably 25 to 100 g/m², particularly 35 to 80 g/m², more particularly 45 to 65 g/m², more preferably of 50 to 60 g/m².

22. Disposable surgical drape according to one of the preceding claims, **characterized in that** the basic drape material (2) is composed of a laminate from one or a plurality of spun-bonded non-woven material and/or melt-blown non-woven material layers, or from a double-layered non-woven/film composite having a non-woven material made from a hydrophilicized spun-bonded non-woven material with a lower side made from a film material.

## Revendications

1. Recouvrement jetable (1, 11, 111) pour champ opératoire comprenant une matériau (2) de recouvrement de base imperméable aux liquides qui présente en utilisation une face inférieure (4) orientée vers le patient et une face supérieure (6), la face supérieure présentant une couche absorbante,
le recouvrement de champ opératoire s'étendant dans le sens de la longueur LR entre une extrémité supérieure (3c) et une extrémité inférieure (3d), la face supérieure (6) présentant une première partie absorbante (8) et au moins une deuxième partie absorbante (10),
la deuxième partie absorbante (10) étant recouverte par une section (12) de matériau dotée de chants de bord (18a, 18b, 18c, 18d, 18e, 18f) et de zones de bord (20a, 20b, 20c, 20d, 20e, 20f) adjacentes aux chants de bord (18a, 18b, 18d, 18e, 18f), qui absorbe les liquides, est imperméable aux liquides et présente une face intérieure (14) et une face extérieure (16), la surface restante de la face supérieure (6) du matériau (2) de recouvrement de base qui n'est pas recouverte par la section (12) de matériau absorbant formant la première partie absorbante (8),
la section (12) de matériau absorbant étant immobilisée de manière non libérable sur toute la surface des zones de bord (20a, 20b, 20c, 20d, 20e, 20f) de la face supérieure (6) du matériau (2) de recouvrement de base et la section (12) de matériau absorbant n'étant essentiellement pas recouverte par le matériau (2) de recouvrement de base dans les zones de bord (20a, 20b, 20c, 20d, 20e, 20f), de telle sorte qu'une poche (24) soit formée entre la section (12) du matériau absorbant et le matériau (2) de recouvrement de base imperméable aux liquides,
au moins certaines parties de l'une des zones de bord (20a, 20b, 20c, 20d, 20e, 20f) de la section (12) de matériau absorbant présentant au moins une barrière (26', 26'') aux liquides, la barrière aux liquides étant formée par un moyen de jonction (22', 22'') qui fixe de manière non libérable la section (12) de matériau absorbant sur la face supérieure (6) du matériau (2) de recouvrement de base,
**caractérisé en ce que**
le matériau (2) de recouvrement de base est formé d'un feutre en une ou plusieurs couches ou d'un composite de feutre et de film,
**en ce que** la section (12) de matériau absorbant est formée par un feutre en une ou plusieurs couches,
**en ce que** la deuxième zone absorbante est formée par l'extension en surface de la section de matériau absorbant et par l'extension en surface, couverte par la section de matériau absorbant, du matériau de recouvrement de base situé par dessus.

2. Recouvrement jetable pour champ opératoire selon la revendication 1, **caractérisé en ce que** le recouvrement de champ opératoire présente au moins une découpe (30) à l'intérieur de la deuxième zone absorbante (10).

3. Recouvrement jetable pour champ opératoire selon la revendication 2, **caractérisé en ce que** la deuxième zone absorbante (10) entoure directement la découpe (30) et est adjacente à cette dernière, et **en ce que** la section (12) de matériau absorbant présente des chants intérieurs de bord (18b) associés aux bords (32) de la découpe (30) et des zones de bord intérieur (20b) ainsi que des chants extérieurs de bord (18a) et des zones extérieures de bord (20a), les chants intérieurs de bord (18b) de la section (12) de matériau absorbant étant en particulier disposés essentiellement à chant par rapport aux bords (32) de la découpe (30).

4. Recouvrement jetable pour champ opératoire selon l'une des revendications précédentes, **caractérisé en ce qu'**une zone inférieure de bord (20d) de la section (12) de matériau présente une barrière (26', 26'') aux liquides, les zones de bord (20e, 20f) de la section (12) de matériau qui se raccordent à une zone inférieure de bord (20d) de la section (12) de matériau présentant une barrière (26', 26'') aux liquides.

5. Recouvrement jetable pour champ opératoire selon l'une des revendications précédentes, **caractérisé en ce que** seules les zones extérieures de bord (20a) de la section (12) de matériau présentent une barrière (26', 26'') aux liquides.

6. Recouvrement jetable pour champ opératoire selon l'une des revendications précédentes, **caractérisé en ce que** différents moyens de jonction (22', 22'') sont utilisés de manière à former une première et au moins une autre barrière (26', 26'') aux liquides.

7. Recouvrement jetable pour champ opératoire selon l'une des revendications précédentes, **caractérisé en ce que** les moyens de jonction (22', 22'') sont sélectionnés dans l'ensemble constitué des adhésifs fondus à chaud, des rubans adhésifs simples ou double face, des emplacements soudés, en particulier des emplacements soudés par ultrasons, des emplacements soudés thermiquement et des emplacements soudés à la calandreuse.

8. Recouvrement jetable pour champ opératoire selon l'une des revendications précédentes, **caractérisé en ce que** le moyen de jonction (22', 22'') est disposé en plan entre la face supérieure (6) du matériau (2) de recouvrement de base et la face intérieure (14) de la section (12) de matériau absorbant.

9. Recouvrement jetable pour champ opératoire selon l'une des revendications précédentes, **caractérisé en ce que** le moyen de jonction (22', 22'') est disposé entre la face supérieure (6) du matériau (2) de recouvrement de base et la face intérieure (14) de la section (12) de matériau absorbant, les relie directement et est fixé au-dessus du chant de bord (18a, 18b, 18c, 18d, 18e, 18f) de la section (12) de matériau absorbant en s'étendant au-delà de cette dernière pour ensuite être rabattu sur sa face supérieure (16).

10. Recouvrement jetable pour champ opératoire selon l'une des revendications précédentes, **caractérisé en ce que** le moyen de jonction (22', 22'') est disposé sur la face supérieure (6) du matériau (2) de recouvrement de base et s'étend au-delà du chant de bord (18a, 18b, 18c, 18d, 18e, 18f) de la section (12) de matériau absorbant pour ensuite être rabattu et fixé sur sa face supérieure (16).

11. Recouvrement jetable pour champ opératoire selon l'une des revendications précédentes, **caractérisé en ce que** le moyen de jonction (22', 22'') est configuré en forme de rigole et **en ce que** les deux ailes (40a, 40b) de la rigole partant du bord de pliage (42) disposé distalement sont fixées sur la face supérieure (6) du matériau (2) de recouvrement de base et sur la face intérieure (14) de la section (12) de matériau absorbant de telle sorte que la rigole s'ouvre en direction de la poche (24).

12. Recouvrement jetable pour champ opératoire selon l'une des revendications précédentes, **caractérisé en ce que** le moyen de jonction (22', 22'') recouvre aussi bien une zone de bord (20a, 20b, 20c, 20d, 20e, 20f) de la face extérieure (16) de la section (12) de matériau absorbant qu'une partie, directement adjacente à celle-ci, de la face supérieure (6) du matériau (2) de recouvrement de base.

13. Recouvrement jetable pour champ opératoire selon la revendication 7, **caractérisé en ce que** les emplacements soudés sont disposés en forme de motifs de soudage, le motif de soudage présentant, partant d'un chant de bord (18a, 18b, 18c, 18d, 18e, 18f) et de la zone de bord (20a, 20b, 20c, 20d, 20e, 20f) qui lui est adjacente un profil dont la profondeur des emplacements de liaison, la densité des emplacements de liaison et/ou l'épaisseur dés emplacements de liaison diminuent en direction de la poche (24).

14. Recouvrement jetable pour champ opératoire selon l'une des revendications précédentes, **caractérisé en ce que** la barrière (26', 26'') aux liquides s'étend en direction de la poche au-delà de la largeur de la zone de bord (20a, 20b, 20c, 20d, 20e, 20f) fixée.

15. Recouvrement jetable pour champ opératoire selon l'une des revendications précédentes, **caractérisé en ce que** les zones de bord (20a, 20b, 20c, 20d, 20e, 20f) fixées occupent une surface qui représente de 2 à 45 %, en particulier de 2 à 35 % et de manière plus particulière de 2 à 30 % de la surface totale de la section (12) de matériau absorbant.

16. Recouvrement jetable pour champ opératoire selon l'une des revendications précédentes, **caractérisé en ce que** la section (12) de matériau absorbant présente une plus grande capacité d'absorption AK que le matériau qui forme la première zone absorbante (8), en particulier **en ce que** la section (12) de matériau absorbant présente une capacité d'absorption AK d'au moins 25 % supérieure, en particulier d'au moins 50 % supérieure, notamment d'au moins 100 % supérieure, en particulier d'au moins 150 % supérieure, en particulier d'au moins 200 % supérieure, en particulier d'au moins 250 % supérieure et de manière tout particulière d'au moins 300 % supérieure que le matériau qui forme la première zone.

17. Recouvrement jetable pour champ opératoire selon l'une des revendications précédentes, **caractérisé en ce que** la section de matériau absorbant présente une capacité relative d'absorption AK d'au moins 150 g/m², en particulier d'au moins 200 g/m², en particulier d'au moins 250 g/m², en particulier d'au moins 300 g/m², en particulier d'au moins 350 g/m², la capacité d'absorption AK étant mesurée selon la méthode décrite.

18. Recouvrement jetable pour champ opératoire selon l'une des revendications précédentes, **caractérisé en ce que** le matériau formant la première zone présente une capacité d'absorption AK de 50 à 250 g/m², en particulier de 80 à 220 g/m², et de manière plus particulière de 100 à 200 g/m², la capacité d'absorption AK étant mesurée selon la méthode décrite.

19. Recouvrement jetable pour champ opératoire selon l'une des revendications précédentes, **caractérisé en ce que** la deuxième zone absorbante (10) présente une extension en surface qui représente de 2 à 65 %, de préférence de 2 à 55 % et de manière plus préférable de 2 à 45 % de la surface totale du matériau de recouvrement de base.

20. Recouvrement jetable pour champ opératoire selon l'une des revendications précédentes, **caractérisé en ce que** la section (12) de matériau absorbant est constituée d'un feutre hydrophilisé présentant une ou plusieurs couches de fibres liées par filage et/ou soufflées à l'état fondu.

21. Recouvrement jetable pour champ opératoire selon l'une des revendications précédentes, **caractérisé en ce que** la section (12) de matériau absorbant présente un poids par unité de surface de préférence de 25 à 100 g/m², en particulier de 35 à 80 g/m², de façon plus particulière de 45 à 65 g/m² et de manière encore plus préférable de 50 à 60 g/m².

22. Recouvrement jetable pour champ opératoire selon l'une des revendications précédentes, **caractérisé en ce que** le matériau (2) de recouvrement de base est constitué d'un stratifié formé d'une ou plusieurs couches de feutre de fibres liées par filage et/ou de feutre de fibres soufflées à l'état fondu ou d'un composite en deux couches de feutre et de film, avec un feutre en fibres liées par filage et hydrophilisées et une face inférieure en un matériau en feuille.
